# EUROPEAN PATENT SPECIFICATION

(11) **EP 1 640 430 B1**
(45) Date of publication and mention of the grant of the patent: **21.09.2011**
(21) Application number: 05014871.7
(22) Date of filing: 08.07.2005
(51) Int. Cl.: H01L 51/50, C09K 11/06, H05B 33/14

(54) **Luminescent material and organic electroluminescent device using the same**
Lumineszenzmaterial und organische elektrolumineszierende Vorrichtung
Matériel luminescent et dispositif électroluminescent organique

(30) Priority: 09.07.2004 JP 2004203268; 06.06.2005 JP 2005165578
(43) Date of publication of application: 29.03.2006
(60) Divisional application: 10151396.8
(73) Proprietor: JNC Corporation, Chiyoda-ku Tokyo (JP)
(72) Inventor: Wang, Guofang, Goikaigan, Ichihara-shi Chiba 290-8551 (JP); Uchida, Manabu, Goikaigan, Ichihara-shi Chiba 290-8551 (JP); Koike, Toshihiro, Goikaigan, Ichihara-shi Chiba 290-8551 (JP)
(74) Representative: HOFFMANN EITLE

(56) References cited:
- EP-A- 1 167 488
- EP-A- 1 191 822
- EP-A- 1 385 221
- US-A- 5 935 721

## Description

### BACKGROUND OF THE INVENTION

### Field of the Invention

The present invention relates to a new luminescent material having an anthracene skeleton, and to an organic electroluminescent (abbreviated to OEL, hereinafter) device using the luminescent material.

### Description of the Related Art

Recently, the OEL device has drawn a great deal of attention as a full-color flat-panel display of the next generation, and the luminescent materials of blue, green and red colors have been actively studied and developed. However, the improvement of the luminescent materials, especially the blue-luminescent materials, is desired. The blue-luminescent materials that have been reported so far include distyrylarylene derivatives like those mentioned in Patent Document 1 (defined later), zinc complexes like those in Patent Document 2, aluminum complexes like those in Patent Document 3, aromatic amine derivatives like those in Patent Document 4, and anthracene derivatives like those in Patent Document 5. Other than Patent Document 5, examples of using anthracene derivatives as luminescent materials are further disclosed in Non-patent Document 1 and Patent Documents 6-8. In Non-patent Document 1, a diphenylanthracene compound is used. However, forming a film with the diphenylanthracene compound is difficult due to its high crystallinity. Patent Documents 6-8 disclose OEL devices that use phenylanthracene derivatives as luminescent materials. In Patent Document 5, an OEL device is disclosed using a naphthalene-substituted anthracene derivative as a luminescent material. However, since each of the above compounds has a symmetric molecular structure, their crystallinities tend to be higher. In Patent Documents 9-12, OEL devices that use compounds having two or more anthracene rings as luminescent materials are proposed to lower the crystallinity and form a high-quality amorphous film. These materials were reported to emit blue-green light.

Moreover, to produce blue OEL devices of high brightness and long lifetime, it has been proposed to dope the luminescent layer with a small amount of fluorescent pigment. For example, Non-patent Document 2 discloses an OEL device that uses a naphthalene-substituted anthracene derivative as a host compound and a perylene derivative as a dopant. Patent Document 13 discloses an OEL device that uses an anthracene derivative as a host compound and an amine-containing styryl derivative as a dopant.

Furthermore, though Patent Document 14 discloses an example that uses a naphthalene-substituted phenylanthracene derivative as a hole-transporting material, the derivative has never been used as a luminescent material.

EP 1 385 221 A2 describes a luminescent compound I(A)(10) with an anthracene skeleton that is shown below.

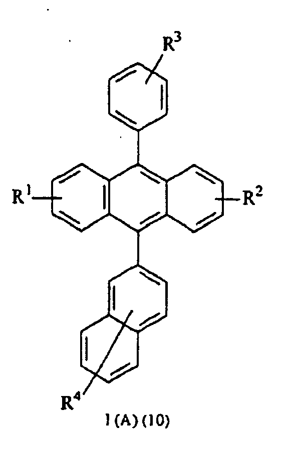

The substituents R¹, R², R³ and R⁴ have various meanings, however, the positions thereof are not specified.
Patent Documents 1-14 and Non-patent Documents 1-2 are defined below.
Patent Document 1: Japanese Patent Publication No. Hei 02-247278
Patent Document 2: Japanese Patent Publication No. Hei 06-336586
Patent Document 3: Japanese Patent Publication No. Hei 05-198378
Patent Document 4: Japanese Patent Publication No. Hei 06-240248
Patent Document 5: Japanese Patent Publication No. Hei 11-3782
Patent Document 6: Japanese Patent Publication No. Hei 11-312588
Patent Document 7: Japanese Patent Publication No. Hei 11-323323
Patent Document 8: Japanese Patent Publication No. Hei 11-329732
Patent Document 9: Japanese Patent Publication No. Hei 8-12600
Patent Document 10: Japanese Patent Publication No. Hei 11-111458
Patent Document 11: Japanese Patent Publication No. 2000-344691
Patent Document 12: Japanese Patent Publication No. 2002-154993
Patent Document 13: PCT Patent Publication No. WO 01/21729
Patent Document 14: Japanese Patent Publication No. 2000-182776
Non-patent Document 1: Applied Physics Letters, 56 (9), 799 (1990)
Non-patent Document 2: Applied Physics Letters, 80 (17), 3201 (2002)

### SUMMARY OF THE INVENTION

This invention is provided in view of the foregoing issues of the prior art. Specifically, one object of this invention is to provide a luminescent material, especially one superior in emission of blue light, which is used in an OEL device and allows the OEL device to have higher luminescence efficiency, lower driving voltage, better thermal resistance and longer lifetime. Another object of this invention is to provide an OEL device that uses the same luminescent material.

This invention is based on the discovery by the inventors that by using a specific compound having an asymmetric structure with a basic anthracene skeleton in the luminescent layer of an OEL device, either alone or in combination with other luminescent material, the OEL device can have higher luminescence efficiency, higher brightness, longer lifetime and lower driving voltage.

The terms used in this invention are defined as follows. An alkyl group means a straight alkyl group or a branched alkyl group, or a group obtained by substituting any methylene group (-CH₂-) of an alkyl group by an oxy moiety (-O-) or an arylene group. The word "any" used in this invention not only means "any position", but also means "any number". Meanwhile, when multiple groups or atoms are to be substituted by other groups, they can be substituted by different groups. For example, when any methylene group of an alkyl group can be substituted by an oxy moiety or an arylene group, the derivative group can be an alkoxyphenyl, alkoxyphenylalkyl, alkoxyalkyl-phenylalkyl, phenoxy, phenylalkoxy, phenylalkoxyalkyl, alkylphenoxy, alkylphenyl-alkoxy or alkylphenylalkoxyalkyl group, etc. Moreover, the alkoxy groups and the alkoxyalkyl groups in the above groups can also be straight groups or branched groups. However, the cases where any methylene group is substituted by an oxy moiety do not include the cases where each of multiple contiguous methylene groups is substituted by an oxy moiety. Moreover, in this specification, "the luminescent material expressed by formula (1)" is often described as "luminescent material (1)" for short, and so on.

The above issues can be solved at least with the following items of this invention. The 1^{st} item is a luminescent material expressed by formula (1): 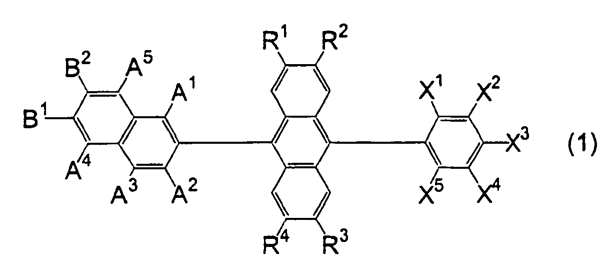 In formula (1), each of R¹-R⁴ represents hydrogen, a C₁-C₂₄ alkyl group or a C₁-C₂₄ alkoxy group. Each of A¹-A⁵ represents hydrogen, a C₁-C₂₄ alkyl group or a C₃-C₂₄ cycloalkyl group. Each of B¹ and B² represents hydrogen, or a C₆-C₂₄ aryl group, wherein B¹ and B² are not simultaneously hydrogen and any hydrogen on the C₆-C₂₄ aryl group can be substituted by a C₁-C₁₂ alkyl group, a C₃-C₁₂ cycloalkyl group or a C₆-C₁₂ aryl group. Each of X¹-X⁵ represents hydrogen, a C₁-C₂₄ alkyl group or a C₃-C₂₄ cycloalkyl group, wherein any methylene group of the C₁-C₂₄ alkyl group can be substituted by an oxy moiety. Any methylene group of the C₁-C₂₄ alkyl group except the one directly bonded to the phenyl group can be substituted by a C₆-C₂₄ arylene group. Any hydrogen on the C₃-C₂₄ cycloalkyl group can be substituted by a C₁-C₂₄ alkyl group or a C₆-C₁₂ aryl group.

The 2^{nd} item is about the luminescent material of the 1^{st} item, wherein each of R¹-R⁴ represents hydrogen, methyl or *t*-butyl. Each of A¹-A⁵ represents hydrogen, methyl, *t*-butyl or cyclohexyl. Each of B¹ and B² represents hydrogen, or is a functional group selected from phenyl, biphenylyl, terphenylyl, quaterphenylyl, naphthyl, phenanthryl, crycenyl and triphenylenyl, on which any hydrogen can be substituted by a C₁-C₁₂ alkyl group, a C₃-C₁₂ cycloalkyl group or a C₆-C₁₂ aryl group. Each of X¹-X⁵ represents hydrogen, a C₁-C₁₂ alkyl group or a C₃-C₁₂ cycloalkyl group. The 3^{rd} item is a luminescent material of the 1^{st} item
wherein in the formula (1), each of R¹-R⁴ represents hydrogen, methyl or *t*-butyl, and each of A¹-A⁵ represents hydrogen. Each of B¹ and B² represents hydrogen, phenyl, 2-biphenylyl, 3-biphenylyl, *m*-terphenyl-5'-yl, *m*-terphenyl-3-yl, 1-naphthyl, 2-naphthyl, 2-(2-naphthyl)phenyl, 3,5-di(1-naphthyl)phenyl, 3,5-di(2-naphthyl)phenyl, *p*-terphenyl-2'-yl, *m*-terphenyl-2-yl, o-terphenyl-2-yl, *p*-terphenyl-2-yl, 5'-phenyl-*m*-terphenyl-2-yl, 5'-phenyl-*m*-terphenyl-3-yl, 5'-phenyl-*m*-terphenyl-3-yl, *m*-quaterphenyl-2-yl, *m*-quaterphenyl-3-yl, 6-(*m*-terphenyl-5'-yl)-2-naphthyl or 4-(*m*-terphenyl-5'-yl)-1-naphthyl. Each of X¹-X⁵ represents hydrogen, methyl, *t*-butyl or cyclohexyl.

The 4^{th} item is about the luminescent material of the above 3^{rd} item, wherein each of X¹-X⁵ represents hydrogen. The 5^{th} item is about the luminescent material of the 3^{rd} item, wherein X³ represents t-butyl and each of X¹, X², X⁴ and X⁵ represents hydrogen. The 6^{th} item is about the luminescent material of the 3^{rd} item, wherein among X¹, X³ and X⁵, at least one represents methyl and the rest represent hydrogen, and each of X² and X⁴ represents hydrogen. The 7^{th} item is about the luminescent material of the 3^{rd} item, wherein each of B¹ and B² represents phenyl, each of X¹ and X⁵ represents hydrogen or methyl, each of X² and X⁴ represents hydrogen, and X³ represents hydrogen, t-butyl or methyl. The 8^{th} item is about the luminescent material of the 3^{rd} item, wherein B¹ represents phenyl, B² represents hydrogen, each of X¹ and X⁵ represents hydrogen or methyl, each of X² and X⁴ represents hydrogen, and X³ represents hydrogen, t-butyl or methyl. The 9^{th} item is also about the luminescent material of the 3^{rd} item, wherein B¹ represents hydrogen, B² represents phenyl, each of X¹ and X⁵ represents hydrogen or methyl, each of X² and X⁴ represents hydrogen, and X³ represents hydrogen, t-butyl or methyl.

The 10^{th} item is about the luminescent material of the 3^{rd} item, wherein each of B¹ and B² represents 2-biphenylyl, each of X¹ and X⁵ represents hydrogen or methyl, each of X² and X⁴ represents hydrogen, and X³ represents hydrogen, t-butyl or methyl. The 11^{th} item is about the luminescent material of the 3^{rd} item, wherein B¹ represents 2-biphenylyl, B² represents hydrogen, each of X¹ and X⁵ represents hydrogen or methyl, each of X² and X⁴ represents hydrogen, and X³ represents hydrogen, t-butyl or methyl. The 12^{th} item is also about the luminescent material of the 3^{rd} item, wherein B¹ represents hydrogen, B² represents 2-biphenylyl, each of X¹ and X⁵ represents hydrogen or methyl, each of X² and X⁴ represents hydrogen, and X³ represents hydrogen, *t*-butyl or methyl.

The 13^{th} item is about the luminescent material of the 3^{rd} item, wherein each of B¹ and B² represents 3-biphenylyl, each of X¹ and X⁵ represents hydrogen or methyl, each of X² and X⁴ represents hydrogen, and X³ represents hydrogen, *t*-butyl or methyl. The 14^{th} item is about the luminescent material of the 3^{rd} item, wherein B¹ represents 3-biphenylyl, B² represents hydrogen, each of X¹ and X⁵ represents hydrogen or methyl, each of X² and X⁴ represents hydrogen, and X³ represents hydrogen, *t*-butyl or methyl. The 15^{th} item is also about the luminescent material of the 3^{rd} item, wherein B¹ represents hydrogen, B² represents 3-biphenylyl, each of X¹ and X⁵ represents hydrogen or methyl, each of X² and X⁴ represents hydrogen, and X³ represents hydrogen, *t*-butyl or methyl.

The 16^{th} item is about the luminescent material of the 3^{rd} item, wherein B¹ represents *m*-terphenyl-5'-yl, B² represents hydrogen, each of X¹ and X⁵ represents hydrogen or methyl, each of X² and X⁴ represents hydrogen, and X³ represents hydrogen, *t*-butyl or methyl. The 17^{th} item is also about the luminescent material of the 3^{rd} item, wherein B¹ represents hydrogen, B² represents *m*-terphenyl-5'-yl, each of X¹ and X⁵ represents hydrogen or methyl, each of X² and X⁴ represents hydrogen, and X³ represents hydrogen, t-butyl or methyl.

The 18^{th} item is about the luminescent material of the 3^{rd} item, wherein B¹ represents *m*-terphenyl-3-yl, B² represents hydrogen, each of X¹ and X⁵ represents hydrogen or methyl, each of X² and X⁴ represents hydrogen, and X³ represents hydrogen, *t*-butyl or methyl. The 19^{th} item is also about the luminescent material of the 3^{rd} item, wherein B¹ represents hydrogen, B² represents *m*-terphenyl-3-yl, each of X¹ and X⁵ represents hydrogen or methyl, each of X² and X⁴ represents hydrogen, and X³ represents hydrogen, t-butyl or methyl.

The 20^{th} item is about the luminescent material of the 3^{rd} item, wherein B¹ represents 1-naphthyl, B² represents hydrogen, each of X¹ and X⁵ represents hydrogen or methyl, each of X² and X⁴ represents hydrogen, and X³ represents hydrogen, *t*-butyl or methyl. The 21^{st} item is also about the luminescent material of the 3^{rd} item, wherein B¹ represents hydrogen, B² represents 1-naphthyl, each of X¹ and X⁵ represents hydrogen or methyl, each of X² and X⁴ represents hydrogen, and X³ represents hydrogen, *t*-butyl or methyl.

The 22^{nd} item is about the luminescent material of the 3^{rd} item, wherein B¹ represents 2-naphthyl, B² represents hydrogen, each of X¹ and X⁵ represents hydrogen or methyl, each of X² and X⁴ represents hydrogen, and X³ represents hydrogen, *t*-butyl or methyl. The 23^{rd} item is also about the luminescent material of the 3^{rd} item, wherein B¹ represents hydrogen, B² represents 2-naphthyl, each of X¹ and X⁵ represents hydrogen or methyl, each of X² and X⁴ represents hydrogen, and X³ represents hydrogen, *t*-butyl or methyl.

The 24^{th} item is about the luminescent material of the 3^{rd} item, wherein B¹ represents 2-(2-naphthyl)phenyl, B² represents hydrogen, each of X¹ and X⁵ represents hydrogen or methyl, each of X² and X⁴ represents hydrogen, and X³ represents hydrogen, t-butyl or methyl. The 25^{th} item is also about the luminescent material of the 3^{rd} item, wherein B¹ represents hydrogen, B² represents 2-(2-naphthyl)phenyl, each of X¹ and X⁵ represents hydrogen or methyl, each of X² and X⁴ represents hydrogen, and X³ represents hydrogen, *t*-butyl or methyl.

The 26^{th} item is about the luminescent material of the 3^{rd} item, wherein B¹ represents 3,5-di(1-naphthyl)phenyl or 3,5-di(2-naphthyl)phenyl, B² represents H, each of X¹ and X⁵ represents hydrogen or methyl, each of X² and X⁴ represents hydrogen, and X³ represents hydrogen, t-butyl or methyl. The 27^{th} item is also about the luminescent material of the 3^{rd} item, wherein B¹ represents hydrogen, B² represents 3,5-di(1-naphthyl)phenyl or 3,5-di(2-naphthyl)phenyl, each of X¹ and X⁵ represents hydrogen or methyl, each of X² and X⁴ represents hydrogen, and X³ represents hydrogen, *t*-butyl or methyl.

The 28^{th} item is about the luminescent material of the 3^{rd} item, wherein B¹ represents a group selected from *p*-terphenyl-2'-yl, *m*-terphenyl-2-yl, *o*-terphenyl-2-yl and *p*-terphenyl-2-yl, B² represents hydrogen, each of X¹ and X⁵ represents hydrogen or methyl, each of X² and X⁴ represents hydrogen, and X³ represents hydrogen, *t*-butyl or methyl. The 29^{th} is also about the luminescent material of the 3^{rd} item, wherein B¹ represents hydrogen, B² represents a group selected from *p*-terphenyl-2'-yl, *m*-terphenyl-2-yl, o-terphenyl-2-yl and *p*-terphenyl-2-yl, each of X¹ and X⁵ represents hydrogen or methyl, each of X² and X⁴ represents hydrogen, and X³ represents hydrogen, *t*-butyl or methyl.

The 30^{th} item is about the luminescent material of the 3^{rd} item, wherein B¹ represents 5'-phenyl-*m*-terphenyl-2-yl or 5'-phenyl-*m*-terphenyl-3-yl, B² represents hydrogen, each of X¹ and X⁵ represents hydrogen or methyl, each of X² and X⁴ represents hydrogen, and X³ represents hydrogen, *t*-butyl or methyl. The 31^{st} item is also about the luminescent material of the 3^{rd} item, wherein B¹ represents hydrogen, B² represents 5'-phenyl-*m*-terphenyl-2-yl or 5'-phenyl-*m*-terphenyl-3-yl, each of X¹ and X⁵ represents hydrogen or methyl, each of X² and X⁴ represents hydrogen, and X³ represents hydrogen, *t*-butyl or methyl.

The 32^{nd} item is about the luminescent material of the 3^{rd} item, wherein B¹ represents *m*-quaterphenyl-2-yl or *m*-quaterphenyl-3-yl, B² represents hydrogen, each of X¹ and X⁵ represents hydrogen or methyl, each of X² and X⁴ represents hydrogen, and X³ represents hydrogen, *t*-butyl or methyl. The 33^{rd} item is also about the luminescent material of the 3^{rd} item, wherein B¹ represents hydrogen, B² represents *m*-quaterphenyl-2-yl or *m*-quaterphenyl-3-yl, each of X¹ and X⁵ represents hydrogen or methyl, each of X² and X⁴ represents hydrogen, and X³ represents hydrogen, *t*-butyl or methyl.

The 34^{th} item is about the luminescent material of the 3^{rd} item, wherein B¹ represents 6-(*m*-terphenyl-5'-yl)-2-naphthyl or 4-(*m*-terphenyl-5'-yl)-1-naphthyl, B² represents hydrogen, each of X¹ and X⁵ represents hydrogen or methyl, each of X² and X⁴ represents hydrogen, and X³ represents hydrogen, *t*-butyl or methyl. The 35^{th} item is also about the luminescent material of the 3^{rd} item, wherein B¹ represents hydrogen, B² represents 6-(*m*-terphenyl-5'-yl)-2-naphthyl or 4-(*m*-terphenyl-5'-yl)-1-naphthyl, each of X¹ and X⁵ represents hydrogen or methyl, each of X² and X⁴ represents hydrogen, and X³ represents hydrogen, *t*-butyl or methyl.

The 36^{th} item is an organic electroluminescent (OEL) device, which includes at least a hole-transporting layer, a luminescent layer and an electron-transporting layer that are sandwiched between an anode and a cathode on a substrate, wherein the luminescent layer includes the luminescent material of any one of the 1^{st} to 35^{th} items of this invention. The 37^{th} item is about the OEL device of the 36^{th} item, wherein the luminescent layer contains at least one luminescent dopant selected from perylene derivatives, borane derivatives, amine-containing styryl derivatives, aromatic amine derivatives, coumarin derivatives, pyran derivatives, iridium complexes and platinum complexes. The 38^{th} item is also about the OEL device of the 36^{th} item, wherein the luminescent layer contains a perylene derivative as a luminescent dopant.

The 39^{th} item is about the OEL device of the 36^{th} item, wherein the luminescent layer contains a borane derivative as a luminescent dopant. The 40^{th} item is about the OEL device of the 36^{th} item, wherein the luminescent layer contains an amine-containing styryl derivative as a luminescent dopant. The 41^{st} item is about the OEL device of the 36^{th} item, wherein the luminescent layer contains an aromatic amine derivative as a luminescent dopant. The 42^{nd} item is about the OEL device of the 36^{th} item, wherein the luminescent layer contains a coumarin derivative as a luminescent dopant. The 43^{rd} item is about the OEL device of the 36^{th} item, wherein the luminescent layer contains a pyran derivative as a luminescent dopant. The 44^{th} item is about the OEL device of the 36^{th} item, wherein the luminescent layer contains an iridium complex as a luminescent dopant. The 45^{th} item is also about the OEL device of the 36^{th} item, wherein the luminescent layer contains a platinum complex as a luminescent dopant.

The 46^{th} item is about the OEL device of any one of the 36^{th} to 45^{th} items, wherein the electron-transporting layer contains a quinolinol metal complex. The 47^{th} item is also about the OEL device of any one of the 36^{th} to 45^{th} items, wherein the electron-transporting layer contains at least one of a pyridine derivative and a phenanthroline derivative.

### Effect of this Invention

Because the luminescent material of this invention has higher fluorescence quantum yield and thermal resistance, it is suitably used, particularly as a host material, in the luminescent layer of an OEL device. Though the luminescent material can be used for emission of various color lights, it is particularly suitable for blue emission. By using the luminescent material, OEL devices with higher luminescence efficiency, lower driving voltage, better thermal resistance and longer lifetime can be obtained. Therefore, a display apparatus that has high performances including full-color display capability can be obtained based on the OEL device of this invention.

### DESCRIPTION OF THE PREFERRED EMBODIMENTS

This invention is explained in more details as follows. The first point of this invention is a luminescent material expressed by formula (1) that has an anthracene skeleton. 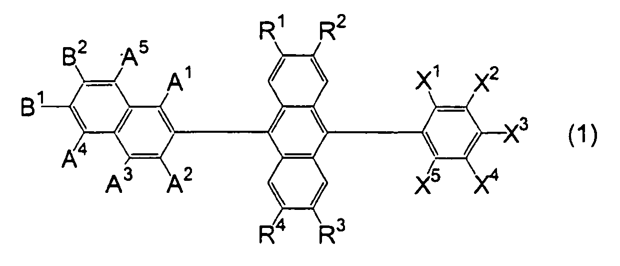 In formula (1), each of R¹-R⁴ represents hydrogen, a C₁-C₂₄ alkyl group or a C₁-C₂₄ alkoxy group. Specific examples of the C₁-C₂₄ alkyl group include methyl, ethyl, *n*-propyl, isopropyl, *n*-butyl, isobutyl, *sec*-butyl, *t*-butyl, *n*-pentyl, isopentyl, *t*-pentyl, neopentyl, *n*-hexyl, isohexyl, 1-methylpentyl, 2-methylpentyl and 5-methylhexyl, etc.

Specific examples of the C₁-C₂₄ alkoxy group include methoxy, ethoxy, propyloxy, isopropyloxy, n-butyloxy, isobutyloxy, sec-butyloxy, t-butyloxy, *n*-pentyloxy, isopentyloxy, *t*-pentyloxy, neopentyloxy, *n*-hexyloxy, isohexyloxy, 1-methylpentyloxy and 2-methylpentyloxy, etc.

Preferred examples of R¹-R⁴ include hydrogen, methyl and t-butyl, wherein hydrogen is particularly preferred.

Each of A¹-A⁵ represents hydrogen, a C₁-C₂₄ alkyl group or a C₃-C₂₄ cycloalkyl group. Specific examples of the C₁-C₂₄ alkyl group include methyl, ethyl, n-propyl, isopropyl, n-butyl, isobutyl, sec-butyl, t-butyl, n-pentyl, isopentyl, t-pentyl, neopentyl, n-hexyl, isohexyl, 1-methylpentyl, 2-methylpentyl and 5-methylhexyl, etc.

Specific examples of the C₃-C₂₄ cycloalkyl group include cyclopropyl, cyclobutyl, cyclopentyl and cyclohexyl, etc.

Preferred examples of A¹-A⁵ include hydrogen, methyl, t-butyl and cyclohexyl, wherein hydrogen is more preferred.

Each of B¹ and B² represents hydrogen or a C₆-C₂₄ aryl group. These groups are described in details as follows.

Firstly, specific examples of the C₆-C₂₄ aryl group include phenyl, 2-biphenylyl, 3-biphenylyl, 4-biphenylyl, *m*-terphenyl-2-yl, *m*-terphenyl-3-yl, *m*-terphenyl-4-yl, *m*-terphenyl-2'-yl, *m*-terphenyl-4'-yl, *m*-terphenyl-5'-yl, *o*-terphenyl-2-yl, *o*-terphenyl-3-yl, *o*-terphenyl-4-yl, *o*-terphenyl-3'-yl, *o*-terphenyl-4'-yl, *p*-terphenyl-2-yl, *p*-terphenyl-3-yl, *p*-terphenyl-4-yl, *p*-terphenyl-2'-yl, *m*-quaterphenyl-2-yl, *m*-quaterphenyl-3-yl, *m*-quaterphenyl-4-yl, *o*-quaterphenyl-2-yl, *o*-quaterphenyl-3-yl, *o*-quaterphenyl-4-yl, *p*-quaterphenyl-2-yl, *p*-quaterphenyl-3-yl, *p*-quaterphenyl-4-yl, 1-naphthyl, 2-naphthyl, 1-phenanthryl, 2-phenanthryl, 3-phenanthryl, 4-phenanthryl, 9-phenanthryl, 1-crycenyl, 2-crycenyl, 3-crycenyl, 5-crycenyl, 6-crycenyl, 1-triphenylenyl and 2-triphenylenyl, etc. Any hydrogen on the C₆-C₂₄ aryl group can be substituted by a C₁-C₁₂ alkyl group, a C₃-C₁₂ cycloalkyl group or a C₆-C₁₂ aryl group.

Specific examples of the C₆-C₂₄ aryl group on which any hydrogen is substituted by a C₁-C₁₂ alkyl group include *o*-tolyl, *m*-tolyl, *p*-tolyl, 2,4-dimethylphenyl, 2,6-dimethylphenyl, 3,5-dimethylphenyl, 2,4,6-trimethylphenyl, 4-*t*-butylphenyl, 2,4-di-*t*-butylphenyl, 2,4,6-tri-*t*-butylphenyl, 2-methyl-4-biphenylyl, 2-methyl-3-biphenylyl, 2-methyl-2-biphenyl, 3,5-di(2'-methylphenyl)phenyl, 3,5-di(3'-methylphenyl)phenyl, 3,5-di(4'-methylphenyl)phenyl, 3,5-di(4'-*t*-butylphenyl)phenyl, 3,5-bis(2',4'-dimethylphenyl)phenyl, 3,5-bis(3',5'-dimethylphenyl)phenyl, 4-methyl-1-naphthyl, 4-*t*-butyl-1-naphthyl, 6-methyl-2-naphthyl and 6-*t*-butyl-2-naphthyl, etc.

Specific examples of the C₆-C₂₄ aryl group on which any hydrogen is substituted by a C₃-C₁₂ cycloalkyl group include: 2-cyclohexylphenyl, 3-cyclohexylphenyl, 4-cyclohexylphenyl, 2,4-dicyclohexylphenyl, 3,5-dicyclohexylphenyl, 4-cyclohexyl-1-naphthyl and 6-cyclohexyl-2-naphthyl, etc.

Specific examples of the C₆-C₂₄ aryl group on which any hydrogen is substituted by a C₆-C₁₂ aryl group include 2-(1-naphthyl)phenyl, 3-(1-naphthyl)phenyl, 4-(1-naphthyl)phenyl, 2-(2-naphthyl)phenyl, 3-(2-naphthyl)phenyl, 4-(2-naphthyl)-phenyl, 3,5-di(1-naphthyl)phenyl, 3,5-di(2-naphthyl)phenyl, 2,4-di(1-naphthyl)phenyl, 2,4-di(2-naphthyl)phenyl, 5-(1-naphthyl)-3-biphenylyl, 5-(2-naphthyl)-3-biphenylyl, 3,5-bis(2-biphenylyl)phenyl, 3,5-bis(3-biphenylyl)phenyl, 3,5-bis(4-biphenylyl)phenyl, 5'-phenyl-*m*-terphenyl-2-yl, 5'-phenyl-*m*-terphenyl-3-yl, 5'-phenyl-*m*-terphenyl-4-yl, 5'-(1-naphthyl)-*m*-terphenyl-2-yl, 5'-(1-naphthyl)-*m*-terphenyl-3-yl, 5'-(1-naphthyl)-*m*-terphenyl-4-yl, 5'-(2-naphthyl)-*m*-terphenyl-2-yl, 5'-(2-naphthyl)-*m*-terphenyl-3-yl, 5'-(2-naphthyl)-*m*-terphenyl-4-yl, 4-phenyl-1-naphthyl, 6-phenyl-2-naphthyl, 2,2'-binaphthyl-6-yl, 1,2'-binaphthyl-6'-yl, 1,2'-binaphthyl-4-yl, 1,1'-binaphthyl-4-yl, 4-(2-biphenylyl)-1-naphthyl, 4-(3-biphenylyl)-1-naphthyl, 4-(4-biphenylyl)-1-naphthyl, 4-(*m*-terphenyl-5'-yl)-1-naphthyl, 6-(2-biphenylyl)-2-naphthyl, 6-(3-biphenylyl)-2-naphthyl, 6-(4-biphenylyl)-2-naphthyl and 6-(*m*-terphenyl-5'-yl)-2-naphthyl, etc.

Preferred examples of B¹ and B² include hydrogen, phenyl, 2-biphenylyl, 3-biphenylyl, 4-biphenylyl, *m*-terphenyl-4'-yl, *m*-terphenyl-5'-yl, *p*-terphenyl-2'-yl, *p*-terphenyl-2-yl, *m*-terphenyl-2-yl, *m*-terphenyl-3-yl, *o*-terphenyl-2-yl, *o*-terphenyl-3-yl, 3,5-di(2-naphthyl)phenyl, 3,5-di(1-naphthyl)phenyl, 1-naphthyl, 2-naphthyl, 4-phenyl-1-naphthyl, 6-phenyl-2-naphthyl, 1,2'-binaphthyl-4-yl, 2,2'-binaphthyl-6-yl, 9-phenanthryl, 2-triphenylenyl, 2-(2-naphthyl)phenyl, 5'-phenyl-*m*-terphenyl-2-yl, 5'-phenyl-*m*-terphenyl-3-yl, *m*-quaterphenylyl-2-yl, *m*-quaterphenylyl-3-yl, 6-(*m*-terphenyl-5'-yl)-2-naphthyl and 4-(*m*-terphenyl-5'-yl)-1-naphthyl, etc., wherein 2-biphenylyl, 3-biphenylyl, *m*-terphenyl-5'-yl, *m*-terphenyl-3-yl, 1-naphthyl and 2-naphthyl are particularly preferred.

B¹ and B² can be both selected from the above groups, or one of B¹ and B² is selected from the above groups and the other is hygrogen. When B¹ or B² is a bulky group, the steric hindrance of the bulky group will shift the luminescence wavelength to the lower side, so that the compound preferably serves as a host material. Moreover, when B¹ or B² is a bulky group, the glass transition temperature (Tg) of the compound is higher and the compound is preferred for the following reasons. When an OEL device is to be fabricated, a low-Tg material tends to gradually crystallize with time so that the luminescence efficiency or stability thereof is possibly changed, while a high-Tg material does not suffer from the time-dependent changes. However, when B¹ and B² both are bulky groups, synthesis of the compound is difficult due to the steric hindrance.

Any of the above-mentioned groups can be introduced as B¹ or B² without much affecting the properties of the compound. However, introducing the group as B¹ is easier in synthesis and lower in cost than as B².

Each of X¹-X⁵ represents hydrogen, a C₁-C₂₄ alkyl group or a C₃-C₂₄ cycloalkyl group. Specific examples of the C₁-C₂₄ alkyl group include methyl, ethyl, n-propyl, isopropyl, n-butyl, isobutyl, sec-butyl, *t*-butyl, *n*-pentyl, isopentyl, *t*-pentyl, neopentyl, *n*-hexyl, isohexyl, 1-methylpentyl, 2-methylpentyl and 5-methylhexyl, etc. Any methylene group of the C₁-C₂₄ alkyl group can be substituted by an oxy moiety. Moreover, any methylene group of the C₁-C₂₄ alkyl group except the one directly bonded to the phenyl group can be substituted by a C₆-C₂₄ arylene group. Examples of the C₆-C₂₄ arylene group can be the same as the aforementioned.

Specific examples of the C₁-C₂₄ alkyl group of which any methylene group is substituted by an oxy moiety include methoxy, ethoxy, propyloxy, isopropyloxy, *n*-butyloxy, isobutyloxy, *sec*-butyloxy, *t*-butyloxy, *n*-pentyloxy, isopentyloxy, *t*-pentyloxy, neopentyloxy, *n*-hexyloxy, isohexyloxy, 1-methylpentyloxy, 2-methylpentyloxy and n-hexyloxy, etc. Specific examples of the C₁-C₂₄ alkyl group of which any methylene group except the one directly bonded to the phenyl group is substituted by a C₆-C₂₄ arylene group include 2-phenylethyl, 2-(4-methylphenyl)ethyl, 1-methyl-1-phenylethyl, 1,1-dimethyl-2-phenylethyl, trityl, 2-(4-biphenylyl)ethyl, 2-(4'-methyl-biphenylyl)ethyl, 2-(4-methyl-1-naphthyl)ethyl and 2-(6-methyl-2-naphthyl)ethyl, etc.

Specific examples of the C₁-C₂₄ alkyl group, of which any methylene group is substituted by an oxy moiety and any methylene group except the one directly bonded to the phenyl group is substituted by a C₆-C₂₄ arylene group, include phenoxy, *o*-tolyloxy, *m*-tolyloxy, *p*-tolyloxy, 1-naphthoxy, 2-naphthoxy, 2,4-dimethylphenoxy, 2,6-dimethylphenoxy, 2,4,6-trimethylphenoxy, 4-*t*-butylphenoxy, 2,4-di-*t*-butylphenoxy, 2,4,6-tri-*t*-butylphenoxy, 2-phenylethoxy and 2-(4-methylphenyl)ethoxy, etc.

Specific examples of the C₃-C₂₄ cycloalkyl group include cyclopropyl, cyclobutyl, cyclopentyl and cyclohexyl, etc. Any hydrogen on the C₃-C₂₄ cycloalkyl group can be substituted by a C₁-C₂₄ alkyl group or a C₆-C₁₂ aryl group.

Specific examples of the C₃-C₂₄ cycloalkyl group on which any hydrogen is substituted by a C₁-C₂₄ alkyl group include 2-methylcyclohexyl, 3-methylcyclohexyl, 4-methylcyclohexyl, 2,4,6-trimethylcyclohexyl, 2-*t*-butylcyclohexyl, 3-*t*-butylcyclohexyl, 4-*t*-butylcyclohexyl and 2,4,6-tri-*t*-butylcyclohexyl, etc. Specific examples of the C₃-C₂₄ cycloalkyl group on which any hydrogen is substituted by a C₆-C₁₂ aryl group include 2-phenylcyclohexyl, 3-phenylcyclohexyl, 4-phenyl- cyclohexyl, 2,4-diphenylcyclohexyl and 3,5-diphenylcyclohexyl, etc.

Preferred examples of X¹-X⁵ include hydrogen, methyl, *t*-butyl and cyclohexyl, wherein hydrogen, methyl and *t*-butyl are more preferred. Preferred examples of the combinations of X¹-X⁵ are shown below together with the phenyl group to which X¹-X⁵ are bonded.

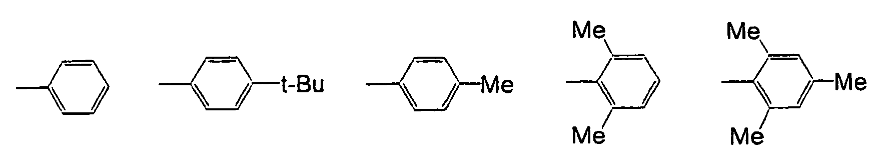

Specific examples of the luminescent material (1) of this invention include the following compounds of formulae (20)-(103). However, the scope of this invention is not limited by the disclosure of these specific molecular structures.

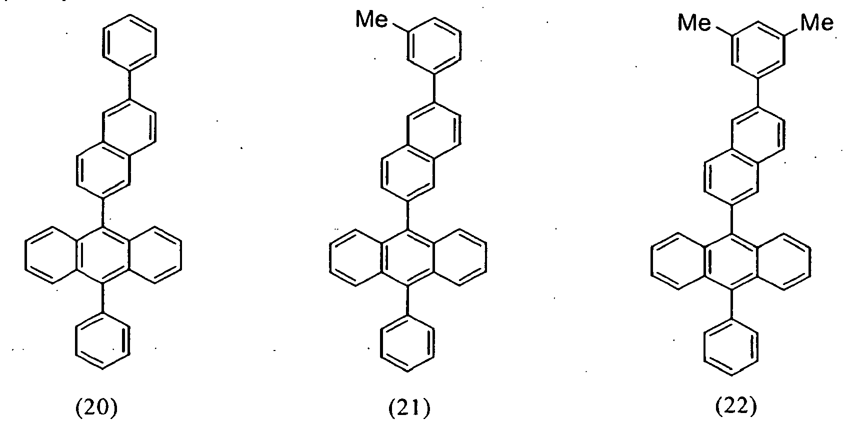

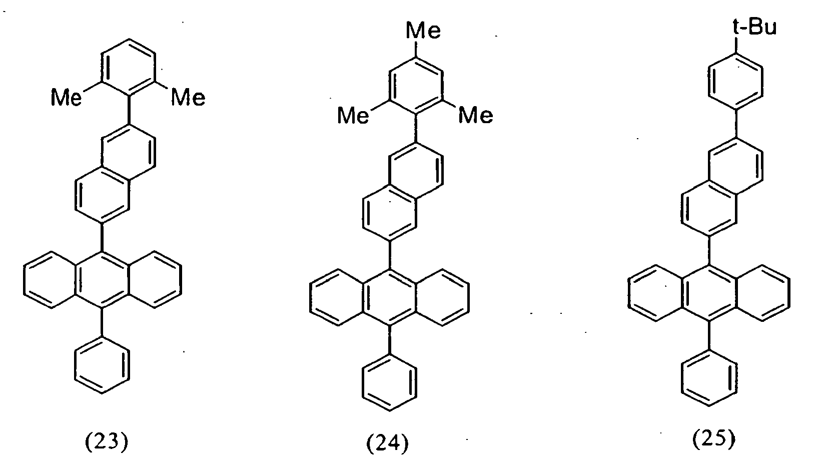

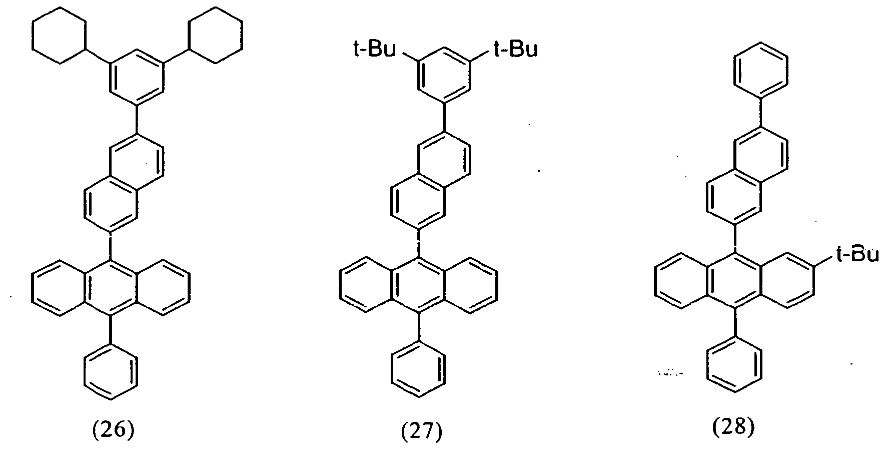

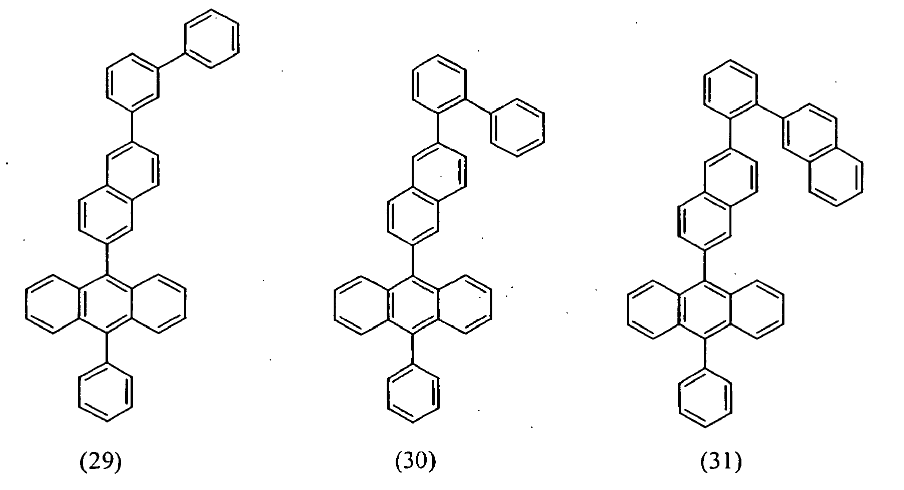

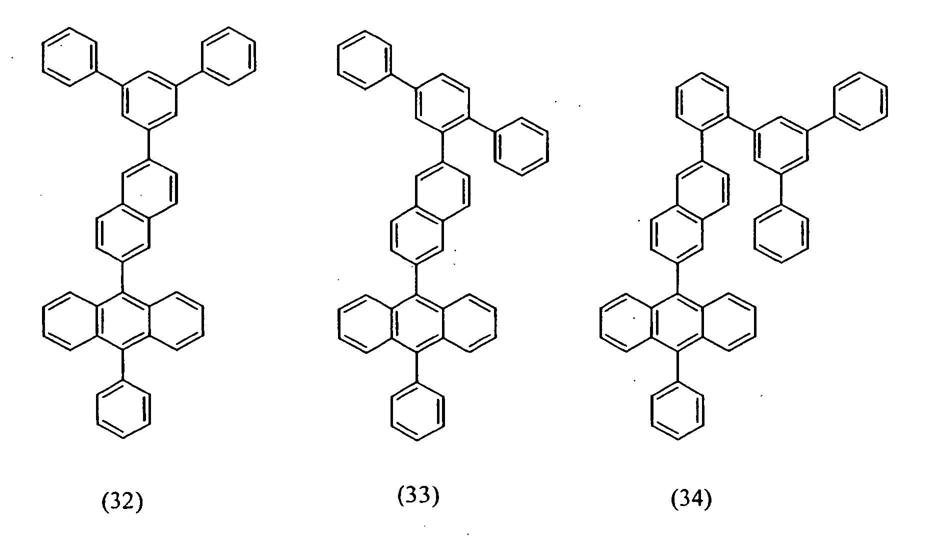

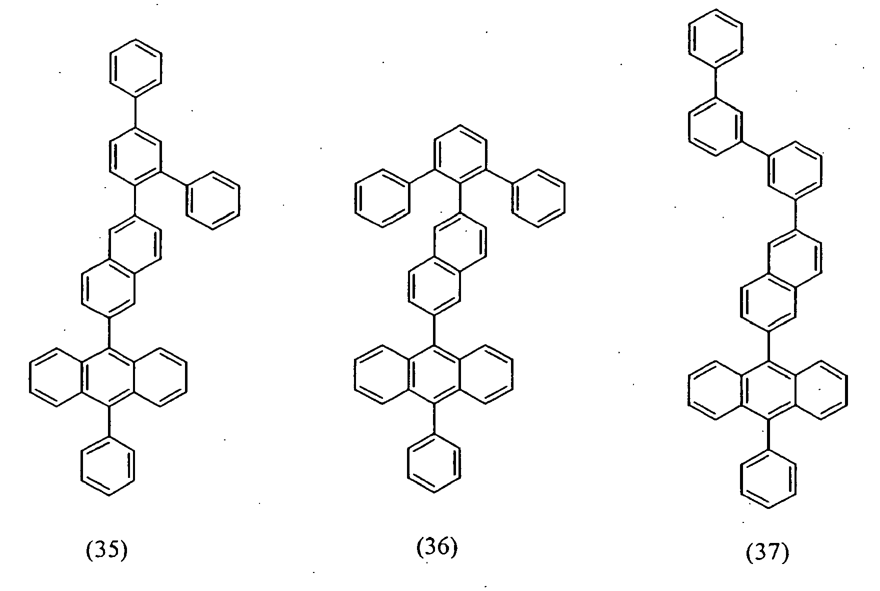

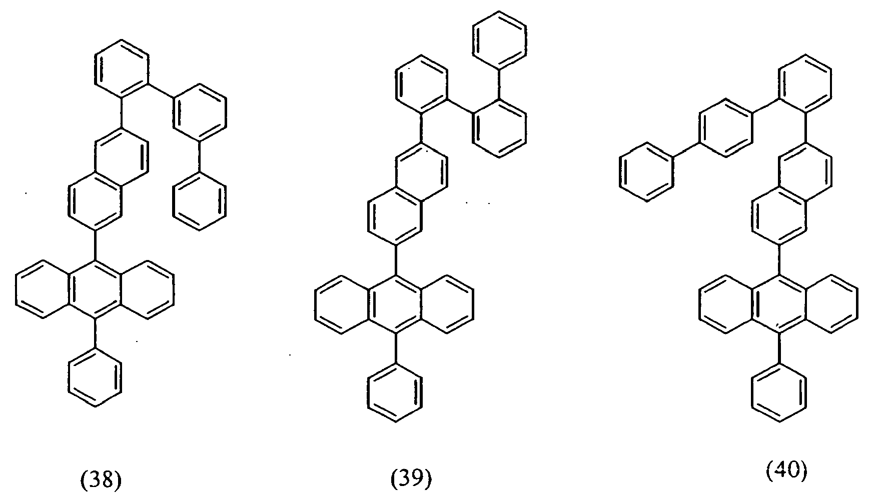

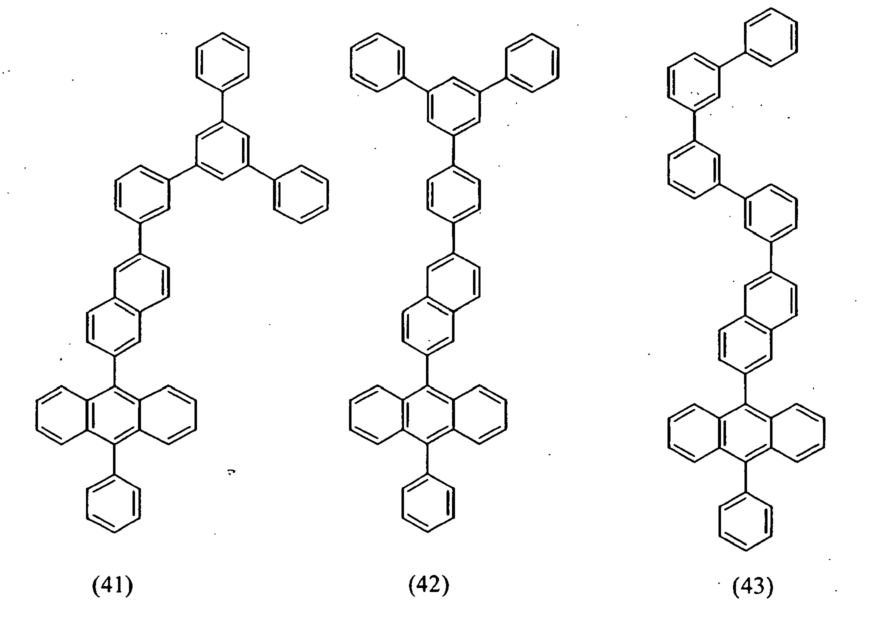

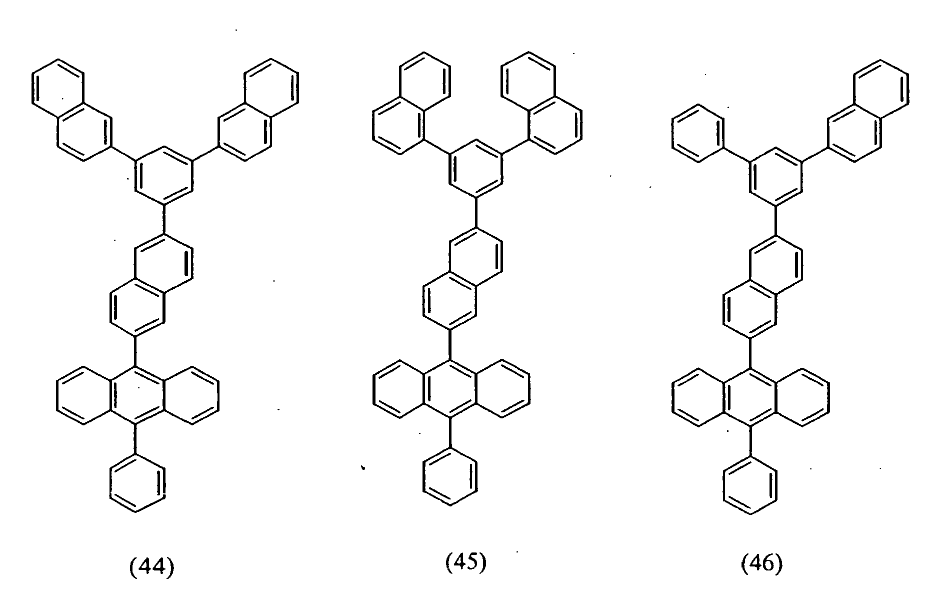

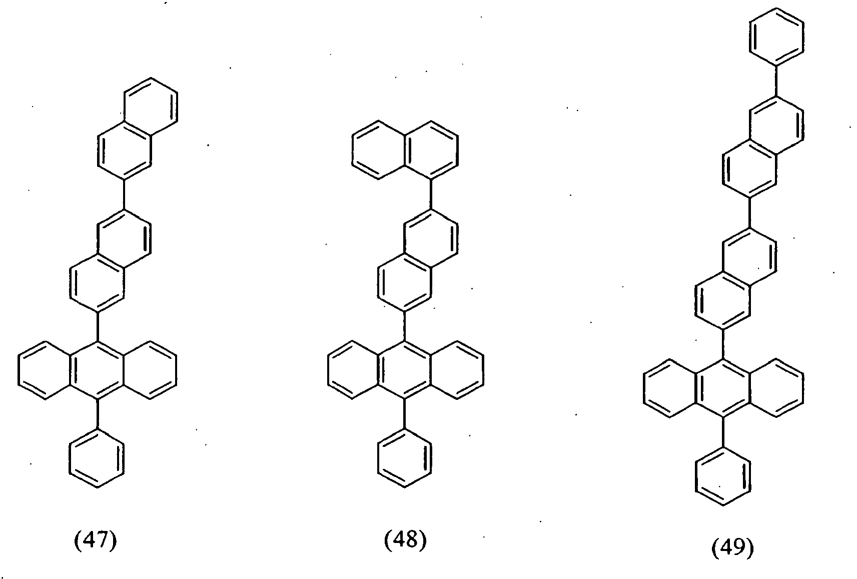

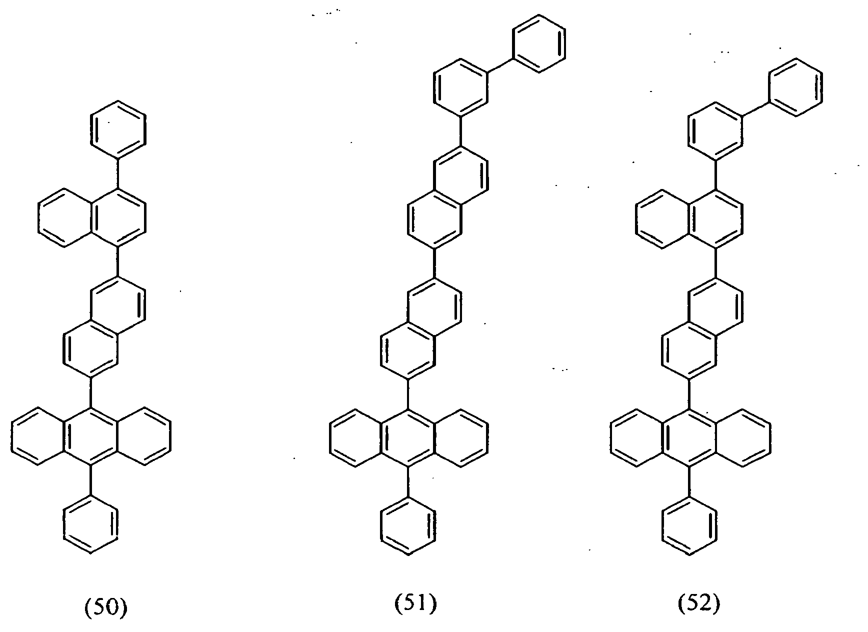

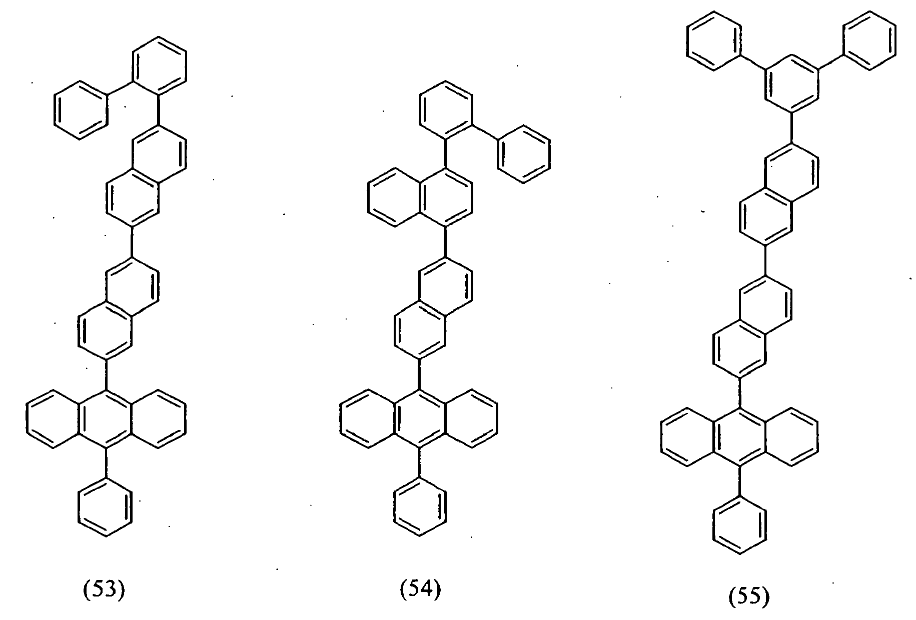

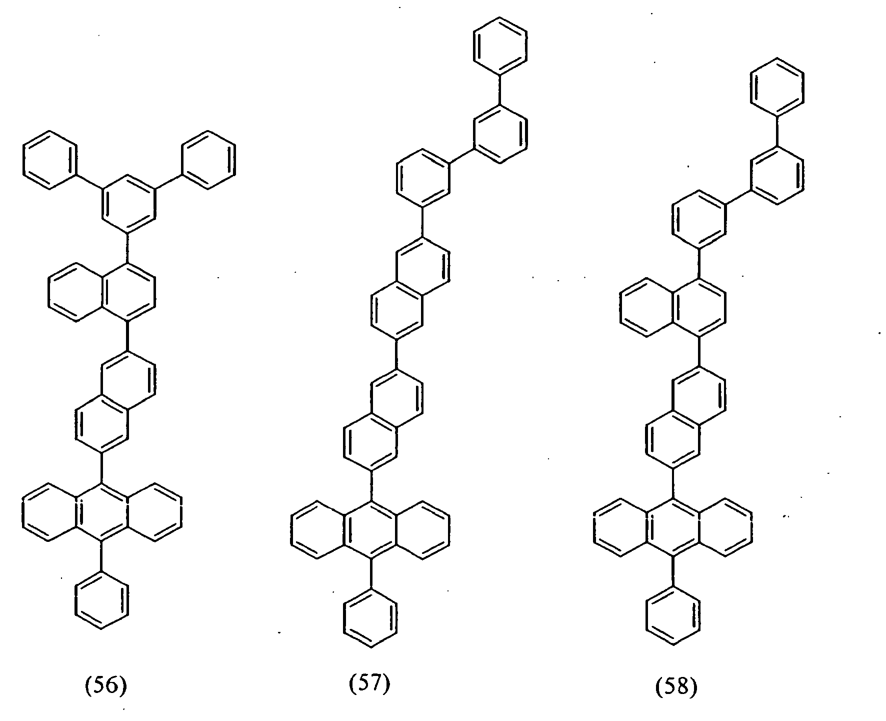

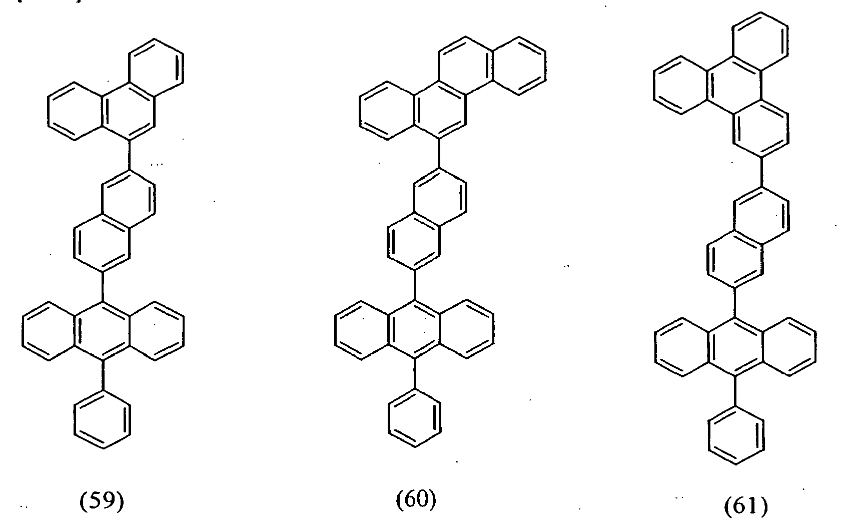

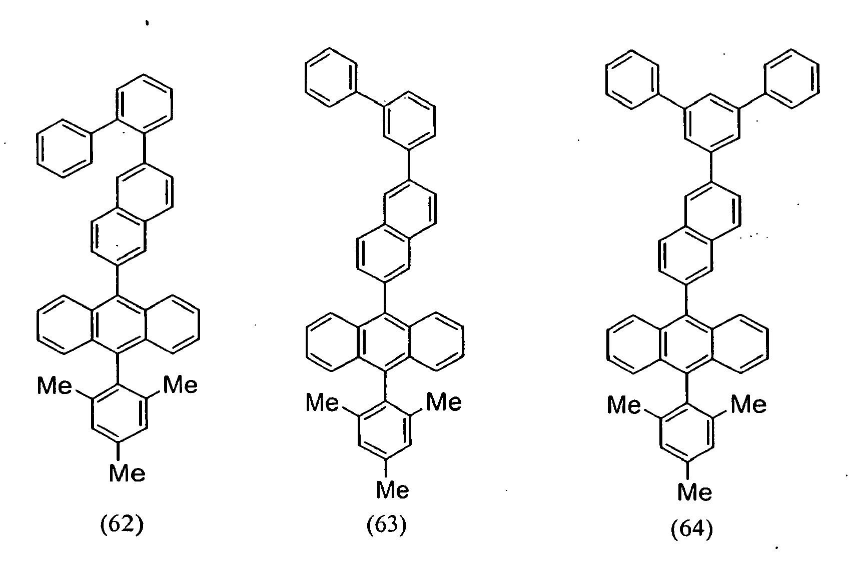

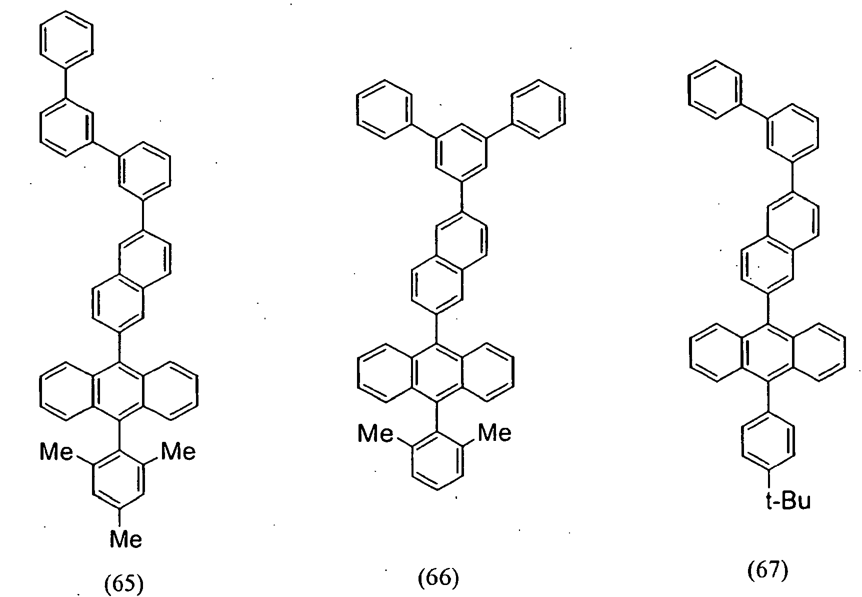

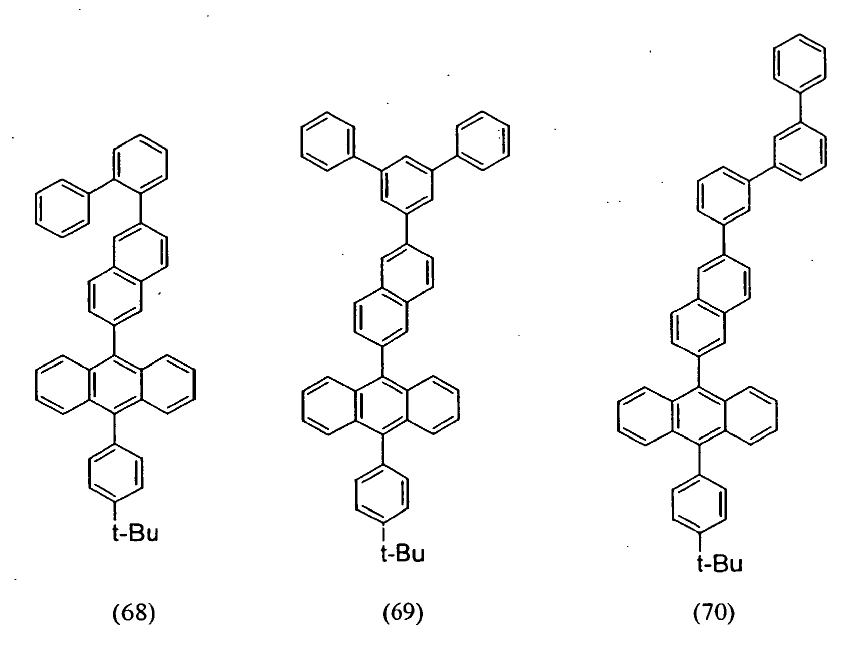

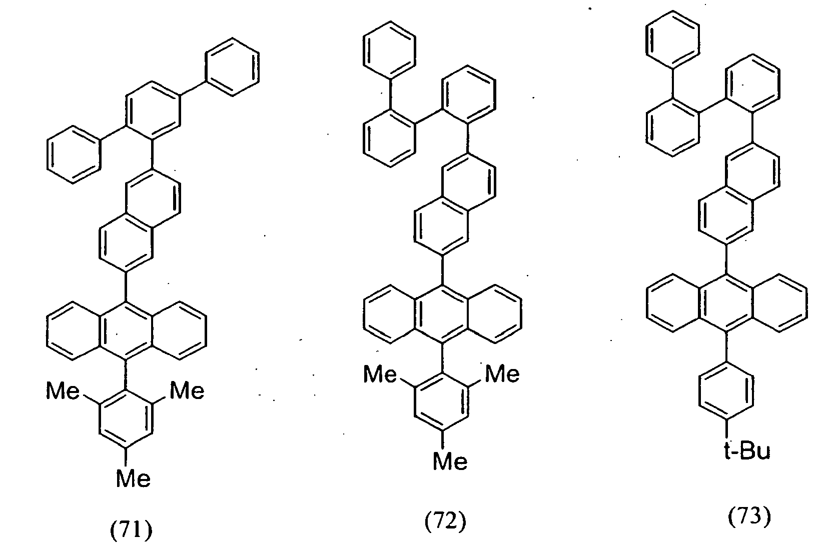

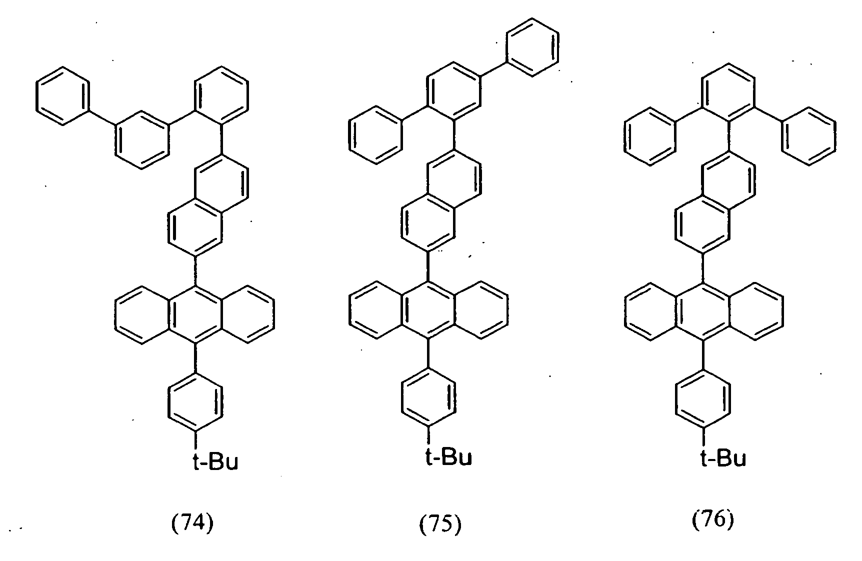

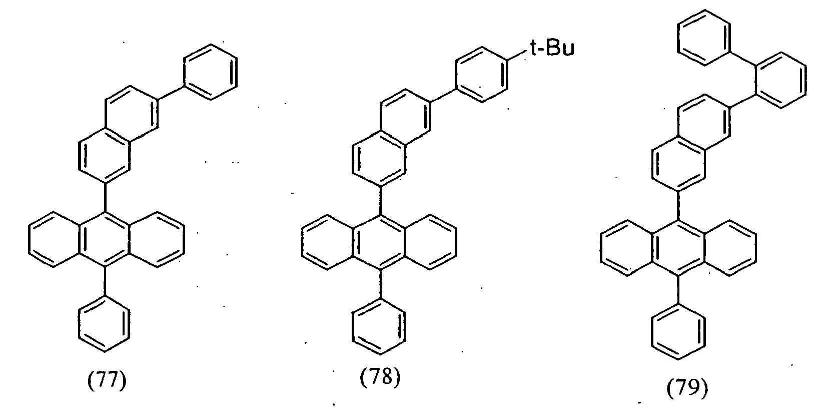

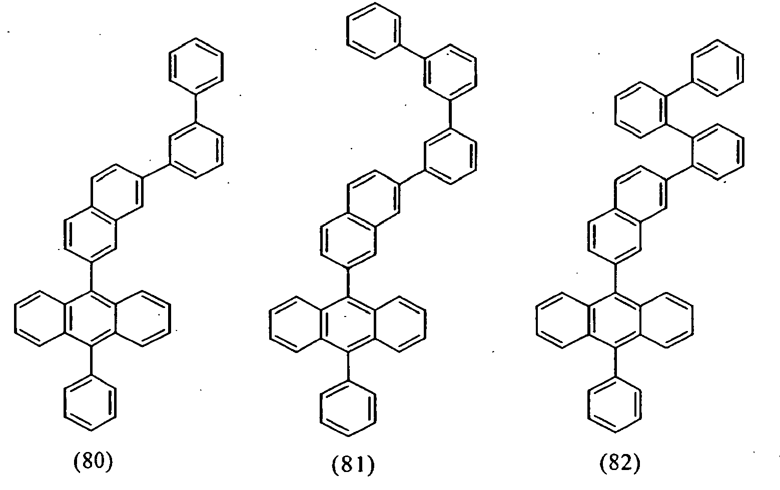

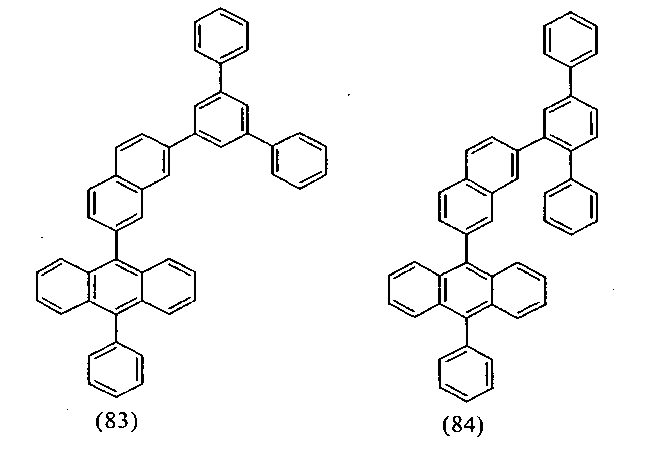

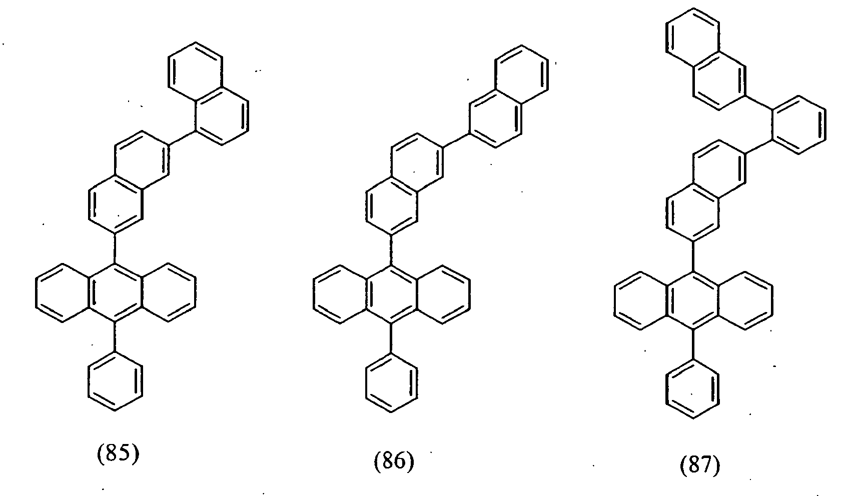

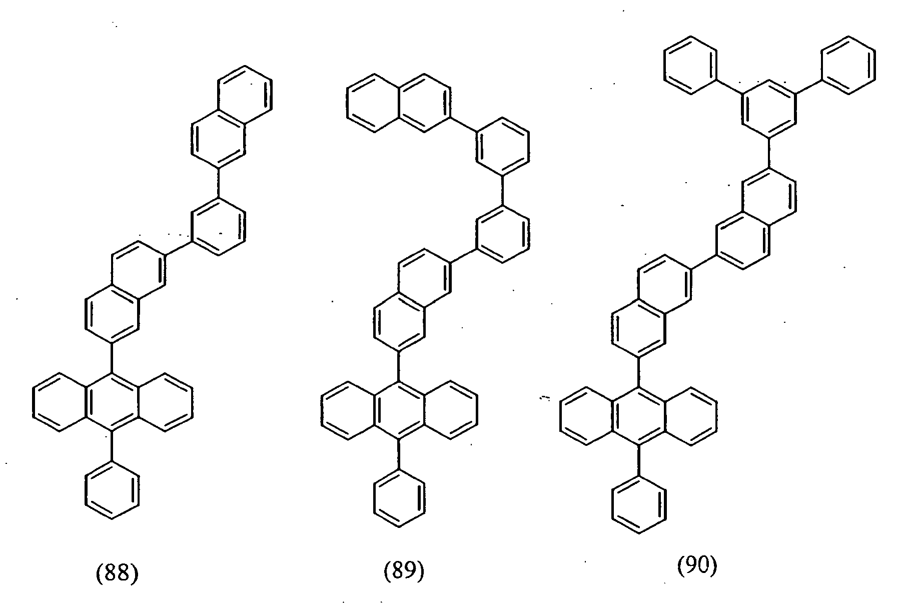

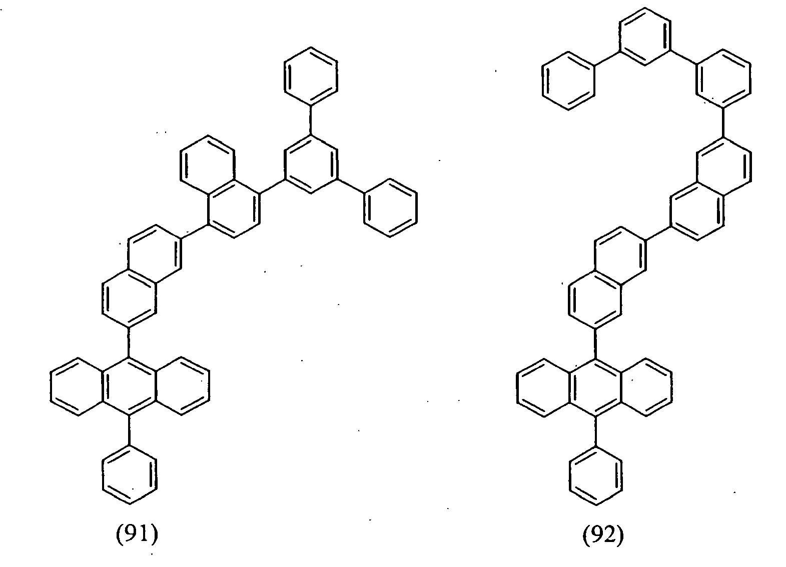

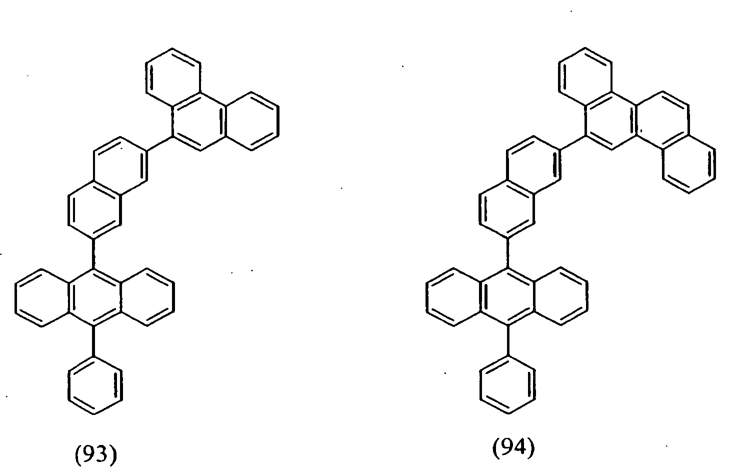

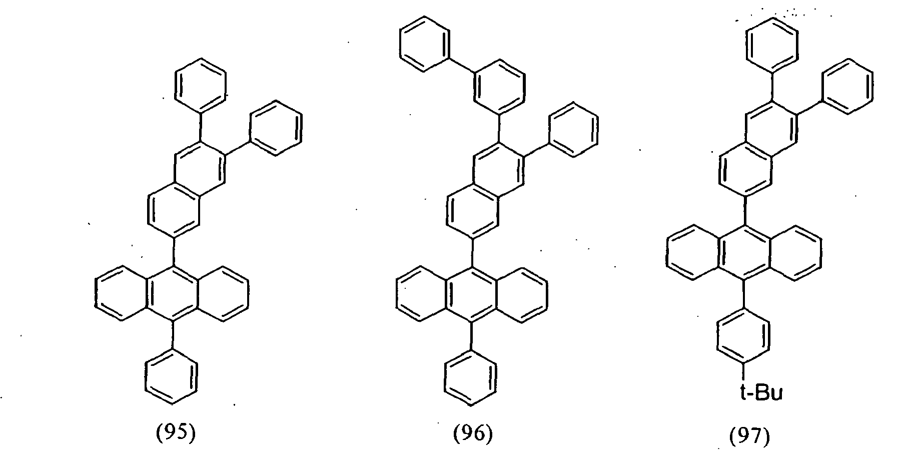

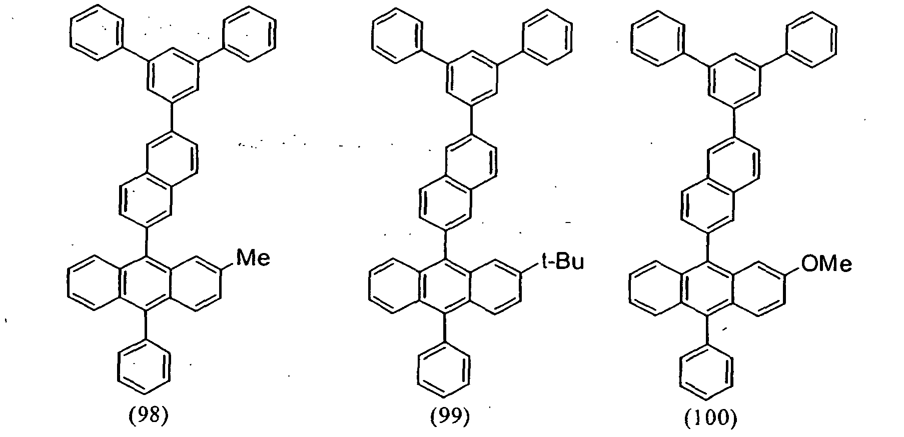

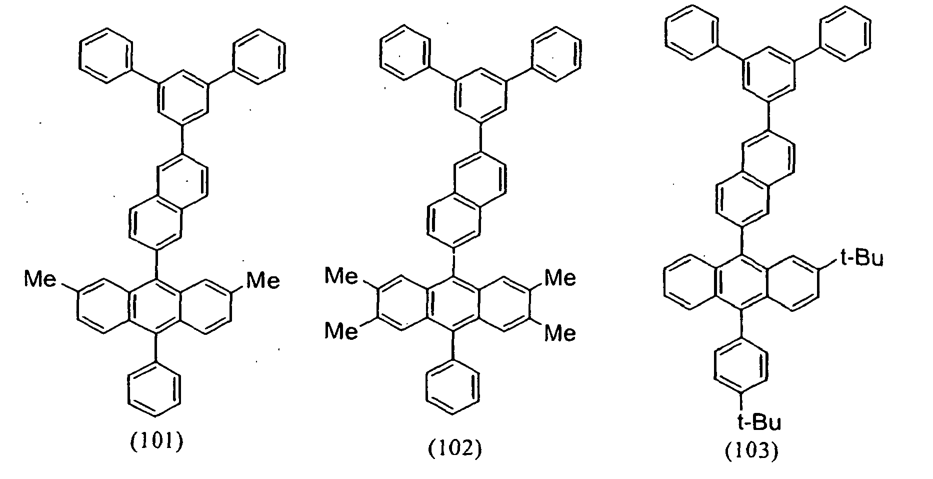

Among the above specific examples, the compounds (20), (29), (30), (31), (32), (33), (34), (37), (38), (39), (40), (41), (43), (44), (45), (47), (48), (55), (56), (64), (69), (70), (71) and (75) are preferred, wherein the compounds (31), (32), (37), (39), (40), (43), (44), (47) and (55) are more preferred.

The luminescent material of this invention can be synthesized with known methods like Suzuki coupling reaction, in which an aromatic halide is coupled with an aromatic boric acid by using a palladium catalyst in presence of a base. Two specific examples of the reaction path for synthesizing the luminescent material (1) through Suzuki coupling reaction are given below: 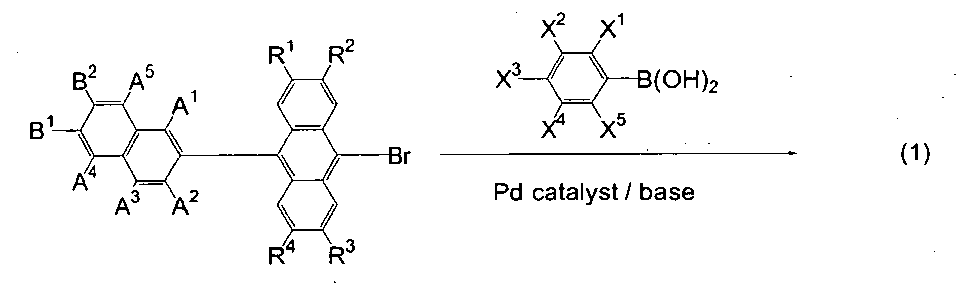 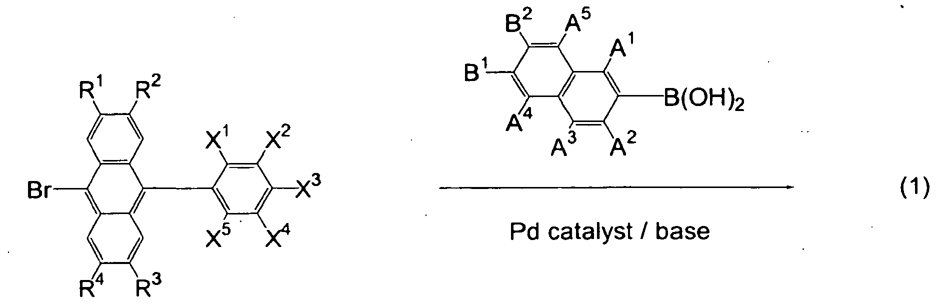 In the above formulae, R¹-R⁴, X¹-X⁵, A¹-A⁵ and B¹ and B² are defined as above.

The specific examples of the palladium catalyst used in the reaction include Pd(PPh₃)₄, PdCl₂(PPh₃)₂, Pd(OAc)₂, tris(dibenzylideneacetone) dipalladium(0) and tris(dibenzylideneacetone) dipalladium chloroform complex(0), etc. If required, a phosphine compound can be added to these palladium compounds to accelerate the reaction. The specific examples of the phosphine compound include tri(*t*-butyl)-phosphine, tricyclohexylphosphine, 1-(N,N-dimethylaminomethyl)-2-(di-*t*-butylphosphine)ferrocene, 1-(N,N-dibutylaminomethyl)-2-(di-*t*-butylphosphine)ferrocene, 1-(methoxymethyl)-2-(di-*t*-butylphosphine)ferrocene, 1,1'-bis(di-*t*-butylphosphine)-ferrocene, 2,2'-bis(di-*t*-butylphosphine)-1,1'-binaphthyl and 2-methoxy-2'-(di-*t*-butylphosphine)-1,1'-binaphthyl, etc. Specific examples of the base used in the above reaction include Na₂CO₃, K₂CO₃, Cs₂CO₃, NaHCO₃, NaOH, KOH, Ba(OH)₂, sodium ethoxide, sodium *t*-butoxide, sodium acetate, Na₃PO4 and potassium fluoride, etc. Moreover, specific examples of the solvent used in the reaction include benzene, toluene, xylene, N,N-dimethylformamide, tetrahydrofuran, diethyl ether, *t*-butyl methyl ether, 1,4-dioxane, methanol, ethanol, and isopropyl alcohol, etc. The reaction solvent can be suitably selected from the above solvents according to the structures of the aromatic halide and the aromatic boric acid used in the reaction. In addition, the above solvents can be used alone or in combination.

The luminescent material of this invention is a compound capable of emitting intense fluorescence at the solid state and can be used for emission of various color lights. However, the luminescent material is particularly suitable for emission of blue light. Since the luminescent material of this invention has an asymmetric molecular structure, it is easily formed in an amorphous state in fabrication of OEL devices. In addition, the luminescent material has good thermal resistance and is stable under application of electric field. For the above reasons, the luminescent material of this invention well serves as a luminescent material of OEL devices.

The luminescent material of this invention is an effective host luminescent material. Though the luminescent material is good as a blue host luminescent material emitting light of short wavelength, it may be used for luminescence of other colors. By using the luminescent material of this invention as a host luminescent material, the energy transfer occurs more efficiently so that EL devices with higher efficiency and longer lifetime can be obtained.

The second point of this invention is an OEL device of which the luminescent layer contains a luminescent material expressed by formula (1) of this invention. The OEL device of this invention not only has higher efficiency and longer lifetime, but also has lower driving voltage and higher durability in preservation and in operation.

The OEL device of this invention can be formed with one of various structures, having typically a multi-layer structure in which a hole-transporting layer, a luminescent layer and an electron-transporting layer are disposed between an anode and a cathode. Specific examples of the multi-layer structure include 1) anode/hole-transporting layer/luminescent layer/electron-transporting layer/cathode, 2) anode/hole-injecting layer/hole-transporting layer/luminescent layer/electron-transporting layer/cathode and 3) anode/hole-injecting layer/hole-transporting layer/luminescent layer/electron-transporting layer/electron-injecting layer/cathode.

Because the luminescent material of this invention has higher luminescence quantum yield and is better in the properties of hole injection, hole transportation, electron injection and electron transportation, it can be used in the luminescent layer effectively. In the OEL device of this invention, the luminescent layer can be formed only from the luminescent material of this invention. However, by combining the luminescent material of this invention with other luminescent material or luminescent dopant material, the OEL device can have higher luminescence brightness or efficiency and may emit blue, green, red or white light. Moreover, when a combined luminescent material is to be used, the luminescent material of this invention may alternatively be used as a luminescent dopant. However, to improve the luminescent efficiency and the lifetime of the OEL devices, the luminescent material of this invention is preferably used together with other luminescent material or luminescent dopant.

Other luminescent materials or luminescent dopants that can be used in the luminescent layer together with the luminescent material of this invention include the daylight fluorescent materials, fluorescent whitening agents, laser pigments, organic scintillators and various fluorescence-analysis reagents described in page 236, Optical Functional Materials (Vol. 6) of the Series of Polymer Functional Materials (edited by the Society of Polymer Science, Japan and co-published in 1991), and the dopant materials and the triplet luminescent materials respectively described in pages 155-156 and pages 170-172, OEL Materials & Display, which is edited under the supervision of Kido Junji and published by CMC Publishing Co. Ltd.

The compounds that can be used as other luminescent materials or luminescent dopants include polycyclic aromatic compounds, heteroaromatic compounds, organometallic complexes, pigments, polymeric luminescent materials, styryl derivatives, aromatic amine derivatives, coumarin derivatives, borane derivatives, oxazine derivatives, compounds containing a spiro-ring, oxadiazole derivatives and fluorene derivatives, etc. Examples of the polycyclic aromatic compounds include anthracene derivatives, phenanthrene derivatives, naphthacene derivatives, pyrene derivatives, chrysene derivatives, perylene derivatives, coronene derivatives and rubrene derivatives, etc. Examples of the heteroaromatic compounds include oxadiazole derivatives having a dialkylamino group or a diarylamino group, pyrazoloquinoline derivatives, pyridine derivatives, pyran derivatives, phenanthroline derivatives, silole derivatives, thiophene derivatives having a triphenylamino group, and quinacridon derivatives, etc. Examples of the organometallic complexes include any combination of the following metal elements with the following organic ligands. The metal elements include Zn, Al, Be, Eu, Tb, Dy, Ir and Pt, etc., and the organic ligands include quinolinol derivatives, benzoxazole derivatives, benzothiazole derivatives, oxadiazole derivatives, thiadiazole derivatives, phenylpyridine derivatives, phenylbenzoimidazole derivatives, pyrrole derivatives, pyridine derivatives and phenanthroline derivatives, etc. Examples of the pigments include xanthene derivatives, polymethine derivatives, porphyrin derivatives, coumarin derivatives, dicyanomethylenepyran derivatives, dicyanomethylenethiopyran derivatives, oxobenzanthracene derivatives, carbostyryl derivatives, perylene derivatives, benzoxazole derivatives, benzothiazole derivatives and benzoimidazole derivatives, etc. Examples of the polymeric luminescent materials include polyparaphenylvinylene derivatives, polythiophene derivatives, polyvinylcarbazole derivatives, polysilane derivatives, polyfluorene derivatives and polyparaphenylene derivatives, etc. Examples of the styryl derivatives include amine-containing styryl derivatives and styrylarylene derivatives, etc.

Particularly, the luminescent dopant is preferably a perylene derivative, a borane derivative, an amine-containing styryl derivative, an aromatic amine derivative, a coumarin derivative, a pyran derivative, an iridium complex or a platinum complex. Specific examples of the perylene derivatives include 3,10-bis(2,6-dimethylphenyl)-perylene, 3,10-bis(2,4,6-trimethylphenyl)perylene, 3,10-diphenylperylene, 3,4-diphenyl-perylene, 2,5,8,11-tetra-*t*-butylperylene, 3,4,9,10-tetraphenylperylene, 3-(1'-pyrenyl)-8,11-di(*t*-butyl)perylene, 3-(9'-anthryl)-8,11-di(*t*-butyl)perylene and 3,3'-bis(8,11-di(*t*-butyl)perylenyl), etc. Specific examples of the borane derivatives include 1,8-diphenyl-10-(dimesitylboryl)anthracene, 9-phenyl-10-(dimesitylboryl)-anthracene, 4-(9'-anthryl)dimesitylborylnaphthalene, 4-(10'-phenyl-9'-anthryl)-dimesitylborylnaphthalene, 9-(dimesitylboryl)anthracene, 9-(4'-biphenylyl)-10-(dimesitylboryl)anthracene and 9-(4'-(N-carbazolyl)phenyl)-10-(dimesitylboryl)-anthracene, etc. Specific examples of the coumarin derivatives include Coumarin-6 and Coumarin-334.

Specific examples of the amine-containing styryl derivatives include N,N,N',N'-tetra(4-biphenylyl)-4,4'-diaminostilbene, N,N,N',N'-tetra(1-naphthyl)-4,4'-diamino-stilbene, N,N,N',N'-tetra(2-naphthyl)-4,4'-diaminostilbene, N,N'-di(2-naphthyl)-N,N'-diphenyl-4,4'-diaminostilbene, N,N'-di(9-phenanthryl)-N,N'-diphenyl-4,4'-diamino-stilbene, 4,4'-bis[4"-bis(diphenylamino)styryl]biphenyl, 1,4-bis[4'-bis(diphenylamino)-styryl]benzene, 2,7-bis[4'-bis(diphenylamino)styryl]-9,9-dimethylfluorene, 4,4'-bis(9-ethyl-3-carbazolevinylene)biphenyl, and 4,4'-bis(9-phenyl-3-carbazolevinylene)-biphenyl, etc.

Specific examples of the aromatic amine derivatives include N,N,N,N-tetraphenylanthracene-9,10-diamine, 9,10-bis(4-diphenylamino-phenyl)anthracene, 9,10-bis(4-di(1-naphthylamino)phenyl)anthracene, 9,10-bis(4-di(2-naphthylamino)-phenyl)anthracene, 10-di-*p*-torylamino-9-(4-di-*p*-torylamino-1-naphthyl)anthracene, 10-diphenylamino-9-(4-diphenylamino-1-naphthyl)anthracene, 10-diphenylamino-9-(6-diphenylamino-2-naphthyl)anthracene, [4-(4-diphenylamino-phenyl)naphthalene-1-yl]diphenylamine, [6-(4-diphenylamino-phenyl)naphthalene-2-yl]diphenylamine, 4,4'-bis[4-diphenylaminonaphthalene-1-yl]biphenyl, 4,4'-bis[6-diphenylamino-naphthalene-2-yl]biphenyl, 4,4"-bis(4-diphenylaminonaphthalene-1-yl)-*p*-terphenyl and 4,4"-bis(6-diphenylaminonaphthalene-2-yl)-*p*-terphenyl, etc.

Specific examples of the pyran derivatives include DCM and DCJTB, etc.

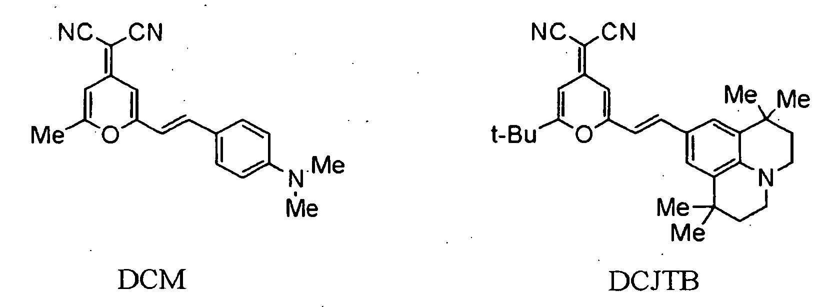

Specific examples of the iridium complexes include Ir(ppy)₃, etc.

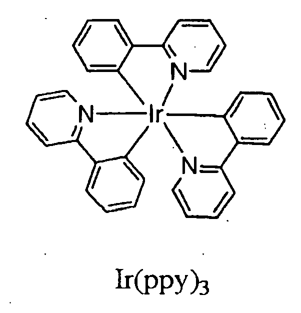

Specific examples of the platinum complexes include PtOEP, etc.

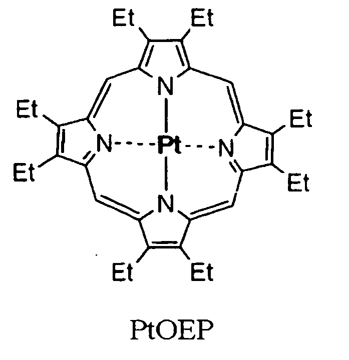

The added amount of the dopant depends on the species thereof, and is preferably determined matching the properties of the dopant. Generally, the amount of the dopant is 0.001-50wt%, preferably 0.1-10wt%, relative to the total amount of the luminescent material.

The electron-transporting material and the electron-injecting material used in the OEL device of this invention can be freely selected from the compounds that can be used as electron-transfer compounds in photoconductive materials and the compounds that can be used in the electron-injecting layer and electron-transporting layer of the prior-art OEL devices.

Specific examples of such electron-transfer compounds include quinolinol metal complexes, pyridine derivatives, phenanthroline derivatives, diphenylquinone derivatives, perylene derivatives, oxadiazole derivatives, thiophene derivatives, triazole derivatives, thiadiazole derivatives, metal complexes of oxine derivatives, quinoxaline derivatives, polymers of quinoxaline derivatives, benzazole compounds, gallium complexes, pyrazole derivatives, perfluorinated phenlylene derivatives, triazine derivatives, pyrazine derivatives, benzoquinoline derivatives, imidazopyridine derivatives and borane derivatives, etc.

Preferred examples of the electron-transfer compounds include quinolinol metal complexes, pyridine derivatives and phenanthroline derivatives. Specific examples of the quinolinol metal complexes include tris(8-hydroxyquinoline) aluminum (abbre. to ALQ, hereinafter), bis(10-hydroxybenzo[h]quinoline) beryllium, tris(4-methyl-8-hydroxyquinoline) aluminum and bis(2-methyl-8-hydroxy-quinoline)-(4-phenylphenol) aluminum, etc. Specific examples of the pyridine derivatives include 2,5-bis(6'-(2',2"-bipyridyl)-1,1-dimethyl-3,4-diphenylsilole (hereinafter as PyPySPyPy), 9,10-di(2',2''-bipyridyl)anthracene, 2,5-di(2',2"-bipyridyl)thiophene, 2,5-di(3',2"-bipyridyl)thiophene and 6',6"-di(2-pyridyl)-2,2':4',3":2",2"'-quaterpyridine, etc. Specific examples of the phenanthroline derivatives include 4,7-diphenyl-1,10-phenarithroline, 2,9-dimethyl-4,7-diphenyl-1,10-phenanthroline, 9,10-di(1,10-phenanthroline-2-yl)anthracene, 2,6-di(1,10-phenanthroline-5-yl)pyridine, 1,3,5-tri(1,10-phenanthroline-5-yl)benzene and 9,9'-difluoro-bis(1,10-phenanthroline-5-yl), etc. Particularly, when a pyridine derivative or phenanthroline derivative is used in the electron-transporting layer or electron-injecting layer, lower driving voltage and higher luminescence efficiency can be achieved.

The hole-transporting material and the hole-injecting material used in the OEL device of this invention can be freely selected from the compounds that are conventionally used as the charge-transporting materials of electron holes in photoconductive materials and the compounds that are known to be used in the hole-injecting layers and hole-transporting layers of conventional OEL devices. Specific examples of such compounds include carbazole derivatives, triarylamine derivatives and phthalocyanine derivatives, etc. Specific examples of the carbazole derivatives include N-phenylcarbazole and polyvinylcarbazole, etc. Specific examples of the triarylamine derivatives include polymers having aromatic tertiary amino groups on the main chain or the side chain, 1,1-bis(4-di-*p*-torylaminophenyl)cyclohexane, N,N'-diphenyl-N,N'-di(3-methylphenyl)-4,4'-diaminobiphenyl, N,N'-diphenyl-N,N'-dinaphthyl-4,4'-diaminobiphenyl (hereinafter as NPD), 4,4',4"-tris[N-(3-methylphenyl)-N-phenylamino]triphenylamine and star-burst amine derivatives, etc. Specific examples of the phthalocyanine derivatives include metal-free phthalocyanine and copper phthalocyanine, etc.

Each of the layers constituting the OEL device of this invention can be formed by making the constituent material of the layer into a film through evaporation deposition, spin cast or a casting method. Though the thickness of each film such formed is not particularly limited and can be suitably set according to the properties of the constituent material, the thickness is usually set in the range of 2nm to 5000nm. Moreover, in order to obtain a uniform film easily and to make pinholes difficult to form, the method for making the luminescent material into a film is preferably evaporation deposition. When evaporation deposition is utilized to make a thin film, the deposition conditions vary with the type of the luminescent material of this invention and the required crystal structure and association structure of the molecular deposition film. Generally, the deposition conditions are preferably set suitably within the following ranges: boat temperature between 50°C and 400°C, vacuum degree between 10⁻⁶ Pa and 10⁻³ Pa, deposition rate between 0.01nm/sec and 50nm/sec, substrate temperature between -150°C and 300°C, and film thickness between 5nm and 5µm.

The OEL device of this invention is preferably supported on a substrate, regardless which of the aforementioned device structures is adopted. The substrate preferably has mechanical strength, thermal stability and transparency, and can be a glass substrate or a transparent plastic film. The anode material can be metal, metal alloy or an electrically conductive compound that has a work function larger than 4eV, or a mixture thereof. Specific examples thereof include some metals like Au, CuI, indium tin oxide (ITO), SnO₂ and ZnO, etc.

The cathode material can be metal, metal alloy or an electrically conductive compound that has a work function smaller than 4eV, or a mixture thereof. Specific examples thereof include Al, Ca, Mg, Li, Mg-alloy and Al-alloy, etc. Specific examples of the metal alloy include Al/LiF, Al/Li, Mg/Ag and Mg/In, etc. To enhance the luminescence efficiency of the OEL device, it is desired that at least one of the anode and cathode has a transparency higher than 10%. The sheet resistance of the electrode is preferably hundreds of ohms per square or lower. Moreover, though the electrode film thickness depends on the properties of the electrode material, it is usually set within the range of 10nm-1µm, preferably within the range of 10-400nm. Such an electrode can be formed as a film through evaporation deposition or sputtering deposition of an above-mentioned electrode material.

An exemplary method for fabricating an OEL device using the luminescent material of this invention is described as follows, wherein the OEL device has the aforementioned structure of anode/hole-injecting layer/hole-transporting layer/luminescent layer (the luminescent material of this invention plus dopant)/electron-transporting layer/cathode. After the anode is made by forming a film of the anode material on a suitable substrate through evaporation deposition, films of the hole-injecting layer and hole-transporting layer are formed on the anode. A luminescent layer is made on the resulting structure as a film formed through co-evaporation deposition of the luminescent material of this invention and a dopant, an electron-transporting layer is formed on the luminescent layer, and then a film of a cathode material is formed through evaporation deposition as a cathode. Thus, the object OEL device is fabricated. Moreover, it is also possible to reverse the above step sequence in the fabrication of the OEL device, that is, to form a cathode, an electron-transporting layer, a luminescent layer, a hole-transporting layer, a hole-injecting layer and an anode in sequence.

Moreover, co-evaporation deposition of the luminescent material and the dopant can be done with a known method. Specifically, the substrate is placed in the upper part of a vacuum chamber, and the two evaporation sources are set in the under part of the vacuum chamber. By evaporating the materials from the two evaporation sources simultaneously, the two materials are mixed and deposited on the substrate. In addition, a partition board is disposed between the two evaporation sources, and film thickness monitors are disposed near the substrate and near each evaporation source. By evaporating the two materials at the same time in constant evaporation rates, a film with a desired mixing ratio can be obtained. Moreover, because there is a partition board between the two evaporation sources, the film-thickness monitor set near one evaporation source does not sense the molecules from the other evaporation source, so that the evaporation rate of each source can be detected using the corresponding monitor. Meanwhile, because the film thickness monitor disposed near the substrate can detect the molecules evaporated from the two evaporation sources, the thickness of the deposited film is detected continuously so that the film thickness on the substrate can be adjusted to the desired one. In this invention, the co-evaporation deposition method is not restricted to that mentioned above, and can be any known method. The principle of the co-evaporation deposition is disclosed, for example, as two-source evaporation deposition in Chapter 9.2 (page 153), 2^{nd} version of *Optical Film* in *Series II of Optical Techniques,* published on October 10, 1986 by Kyoritsu Shuppan Co., Ltd. In addition, the scheme of a practical evaporation apparatus is disclosed, for example, as an evaporation deposition apparatus of organic polymer in Chapter 1.1, Part 3 (FIG. 8 in page 125) of the enlarged and revised edition of Manual of Light/Film Techniques, published on August 31, 1992 by Optronics Co., Ltd. Moreover, Japanese Patent Publication No. 2002-76027 discloses a method of fabricating a coevaporation-deposited organic film. In addition, the application in fabrication of OEL devices is disclosed in, for example, C. W. Tang, S. A. VanSlyke, and C. H. Chen, J. Appl. Phys., 65(9), 3610-3616, (1989).

When the OEL device such obtained is applied with a DC voltage, the anode and the cathode are preferably coupled to positive and negative polarity, respectively, and light emission can be observed from the side(s) of the transparent or semi-transparent electrode(s), i.e., the side of the anode or cathode or the sides of both, by applying a voltage of 2-40V. Moreover, the OEL device also emits light when applied with an AC voltage, wherein the AC voltage can have any waveform. This invention will be explained in more details based on the following Examples.

### Example 1

Synthesis of Compound (32)

In a nitrogen ambient, 3.33g of 9-bromo-10-phenylanthracene and 4.8g of 6-(*m*-terphenyl-5'-yl)naphthalene-2-boric acid are dissolved in 100ml of a mixed solvent of toluene and ethanol in a weight ratio of 4:1, and then 0.58g of tetrakis(triphenylphosphine)palladium(0) is added. The solution is stirred for 5 minutes, and then 10ml of 2M aqueous solution of sodium carbonate is added, followed by reflux of 8 hours. After the reflux, the reaction solution is cooled. The organic layer is separated and collected, washed with saturated NaCl solution, and then dried with anhydrous magnesium sulfate. After the drying agent is removed, the solvent is removed through low-pressure distillation to obtain a solid material, which is purified with a silica gel column and a solvent of heptane and toluene in a ratio of 3:1 and then purified through sublimation to obtain 3.7g of the target compound (32). The structure of compound (32) is confirmed with mass spectrum and NMR, and the physical properties of the same are listed as follows:
Melting point: 280°C
Glass-transition temperature (Tg): 143°C (measuring apparatus: Diamond DSC, made by PERKIN-ELMER Company; measurement conditions: cooling rate = 200°C/min, and heating rate = 10°C/min)
Fluorescence quantum yield (in toluene solution): 0.82 (measuring apparatuses: V-560 and FP-777W, made by JASCO Corporation)

### Example 2

Synthesis of Compound (69)

In a nitrogen ambient, 3.89g of 9-bromo-10-(*t*-butylphenyl)anthracene and 4.8g of 6-(*m*-terphenyl-5'-yl)naphthalene-2-boric acid are dissolved in 100ml of a mixed solvent of toluene and ethanol in a weight ratio of 4:1, and then 0.58g of tetrakis(triphenylphosphine)palladium(0) is added. The solution is stirred for 5 minutes, and then 10ml of 2M aqueous solution of sodium carbonate is added, followed by reflux of 8 hours. After the reflux, the reaction solution is cooled. The organic layer is separated and collected, washed with saturated NaCl solution, and then dried with anhydrous magnesium sulfate. After the drying agent is removed, the solvent is removed through low-pressure distillation to obtain a solid material, which is purified with a silica gel column and a solvent of heptane and toluene in a ratio of 3:1 and then purified through sublimation to obtain 3.0g of the target compound (69). The structure of the compound (69) is confirmed with mass spectrum and NMR, and the physical properties of the same are listed as follows:
Melting point: 300°C
Glass-transition temperature (Tg): 162°C (measuring apparatus: Diamond DSC, made by PERKIN-ELMER Company; measurement conditions: cooling rate = 200°C/min, and heating rate = 10°C/min)

### Example 3

Synthesis of Compound (40)

In a nitrogen ambient, 4.35g of 2-(6-bromo-naphthalene-2-yl)-[1,1';4',1"]-terphenyl and 4.5g of 10-phenyl-anthracene-9-boric acid are dissolved in 50ml of a mixed solvent of toluene and ethanol in a weight ratio of 4:1, and then 0.34g of tetrakis(triphenylphosphine)palladium(0) is added. The solution is stirred for 5 minutes, and then 10ml of 2M aqueous solution of sodium carbonate is added, followed by reflux of 10 hours. After the reflux, the reaction solution is cooled. The organic layer is separated and collected, washed with saturated NaCl solution, and then dried with anhydrous magnesium sulfate. After the drying agent is removed, the solvent is removed through low-pressure distillation to obtain a solid material, which is purified with a silica gel column and a solvent of heptane and toluene in a ratio of 3:1 and then purified through sublimation to obtain 4.9g of the target compound (40). The structure of the compound (40) is confirmed with mass spectrum and NMR, and the physical properties of the same are listed as follows:
Melting point: 305°C
Glass-transition temperature (Tg): 147°C (measuring apparatus: Diamond DSC, made by PERKIN-ELMER Company; measurement conditions: cooling rate = 200°C/min, and heating rate = 10°C/min)

By selecting suitable raw-material compounds, the other luminescent materials of this invention can be readily synthesized based on the method mentioned in the above Synthesis Examples.

### Example 4

A glass substrate of 26mm×28mm×0.7mm having been deposited with ITO of 150nm in thickness through evaporation deposition, which was produced by Tokyo Sanyo Vacuum Industries Co., Ltd., was provided as a transparent support substrate. The transparent support substrate is fixed by the substrate holder of a commercially available evaporation deposition apparatus, which was made by ULVAC KIKO, Inc. Molybdenum evaporation boats loaded with copper phthalocyanine, NPD, the compound (32), ALQ and LiF, respectively, as well as a tungsten evaporation boat loaded with Al, were installed to the apparatus. The pressure in the vacuum chamber was reduced to 1×10⁻³ Pa, and the evaporation boat loaded with copper phthalocyanine is heated to evaporate and deposit copper phthalocyanine to a thickness of 20nm to form a hole-injecting layer. Next, the evaporation boat loaded with NPD is heated to evaporate and deposit NPD to a thickness of 30nm to form a hole-transporting layer. The evaporation boat loaded with compound (32) is then heated to evaporate and deposit the same to a thickness of 35nm to form a luminescent layer. Subsequently, the evaporation boat loaded with ALQ is heated to evaporate and deposit ALQ to a thickness of 15nm to form an electron-transporting layer. The evaporation rates in the above steps were between 0.1nm/sec and 0.2nm/sec. Thereafter, the evaporation boat loaded with LiF is heated to evaporate and deposit LiF to a thickness of 0.5nm in a rate of 0.003-0.01nm/sec, and then the evaporation boat loaded with Al is heated to evaporate and deposit A1 to a thickness of 100nm in a rate of 0.2-0.5nm/sec. Thus, an OEL device was obtained. The ITO electrode served as an anode and the LiF/Al electrode as a cathode, while a DC voltage of about 4.6V was applied between them to produce a current of about 4mA/cm² and cause emission of blue light of 438nm in a luminescence efficiency of 31m/W. Moreover, when the OEL device was driven with a constant current of 50mA/cm², the initial brightness was 1200cd/m², and the life property of the same was indicated by the brightness half-decay time of 300 hours.

### Example 5

An OEL device was obtained with the same method in Example 4, except that the compound (32) was replaced by the compound (69). The ITO electrode served as an anode and the LiF/Al electrode as a cathode, while a DC voltage of about 4.8V was applied between them to produce a current of about 4.2mA/cm² and cause emission of blue light of 439nm in a luminescence efficiency of2.51m/W. Moreover, when the OEL device was driven with a constant current of 50mA/cm², the initial brightness was 1100cd/m², and the life property of the same was indicated by the brightness half-decay time of 260 hours.

### Example 6

An OEL device was obtained with the same method in Example 4, except that ALQ used as the material of the electron-transporting layer in Example 4 was replaced by PyPySPyPy. The ITO electrode served as an anode and the LiF/Al electrode as a cathode, while a DC voltage of about 3V was applied between them to produce a current of about 3mA/cm² and cause emission of blue light of 442nm in a luminescence efficiency of 3.81m/W. Moreover, when the OEL device was driven with a constant current of 50mA/cm², the initial brightness was 1700cd/m², and the life property of the same was indicated by the brightness half-decay time of 200 hours.

### Example 7

A glass substrate of 26mm×28mm×0.7mm having been deposited with ITO of 150nm in thickness through evaporation deposition, which was produced by Tokyo Sanyo Vacuum Industries Co., Ltd., was provided as a transparent support substrate. The transparent support substrate is fixed by the substrate holder of a commercially available evaporation deposition apparatus, which was made by ULVAC KIKO, Inc. Molybdenum evaporation boats loaded with copper phthalocyanine, NPD, the compound (32), 3,10-bis(2,6-dimethylphenyl)perylene, ALQ and LiF, respectively, as well as a tungsten evaporation boat loaded with Al, were installed to the apparatus. The pressure in the vacuum chamber was reduced to 1×10⁻³Pa, and the evaporation boat loaded with copper phthalocyanine is heated to evaporate and deposit copper phthalocyanine to a thickness of 20nm to form a hole-injecting layer. Next, the evaporation boat loaded with NPD is heated to evaporate and deposit NPD to a thickness of 30nm to form a hole-transporting layer. The evaporation boat loaded with compound (32) and that loaded with 3,10-bis(2,6-dimethylphenyl)perylene are then heated to evaporate and deposit the two compounds together to a thickness of 35nm to form a luminescent layer, wherein the doping concentration of 3,10-bis(2,6-dimethylphenyl)perylene is about 1wt%. Subsequently, the evaporation boat loaded with ALQ is heated to evaporate and deposit ALQ to a thickness of 15nm to form an electron-transporting layer. The evaporation rates in the above steps were between 0.1nm/sec and 0.2nm/sec. Thereafter, the evaporation boat loaded with LiF is heated to evaporate and deposit LiF to a thickness of 0.5nm in a rate of 0.003-0.01nm/sec, and then the evaporation boat loaded with Al is heated to evaporate and deposit Al to a thickness of 100nm in a rate of 0.2-0.5nm/sec. Thus, an OEL device was obtained. The ITO electrode served as an anode and the LiF/Al electrode as a cathode, while a DC voltage of about 4.5V was applied between them to produce a current of about 1.5mA/cm² and cause emission of blue light of 468nm in a luminescence efficiency of 4.51m/W. Moreover, when the OEL device was driven with a constant current of 50mA/cm², the initial brightness was 2000cd/m², and the life property of the same was indicated by the brightness half-decay time of 500 hours.

### Example 8

An OEL device was obtained with the same method in Example 7, except that 3,10-bis(2,6-dimethylphenyl)perylene used as a luminescent dopant in Example 7 was replaced by an amine-containing styryl derivative expressed by formula (104) below. The ITO electrode served as an anode and the LiF/Al electrode as a cathode, while a DC voltage of about 4.3V was applied between them to produce a current of about 1.5mA/cm² and cause emission of blue light of 455nm in a luminescence efficiency of 4.81m/W. Moreover, when the OEL device was driven with a constant current of 50mA/cm², the initial brightness was 3100cd/m², and the life property of the same was indicated by the brightness half-decay time of 600 hours.

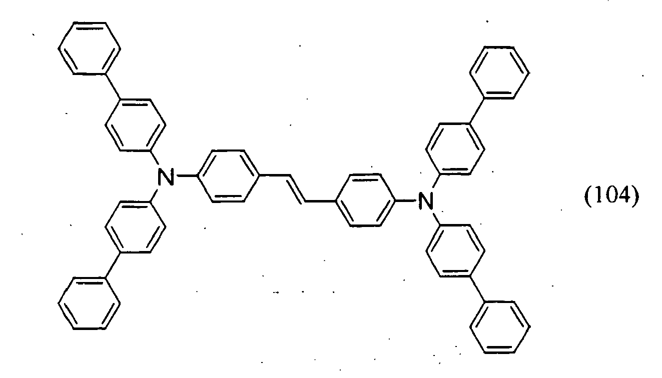

### Example 9

An OEL device was obtained with the same method in Example 7, except that 3,10-bis(2,6-dimethylphenyl)perylene used as a luminescent dopant in Example 7 was replaced by an aromatic amine derivative expressed by formula (105) below. The ITO electrode served as an anode and the LiF/Al electrode as a cathode, while a DC voltage of about 5V was applied between them to produce a current of about 4.1mA/cm² and cause emission of blue light of 447nm in a luminescence efficiency of 3.21m/W. Moreover, when the OEL device was driven with a constant current of 50mA/cm², the initial brightness was 1800cd/m², and the life property of the same was indicated by the brightness half-decay time of 250 hours.

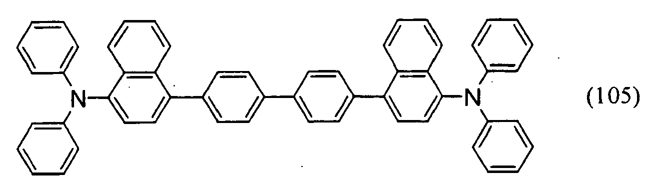

### Example 10

An OEL device was obtained with the same method in Example 7, except that ALQ used as the material of the electron-transporting layer in Example 7 was replaced by PyPySPyPy. The ITO electrode served as an anode and the LiF/Al electrode as a cathode, while a DC voltage of about 2.8V was applied between them to produce a current of about 1mA/cm² and cause emission of blue light of 468nm in a luminescence efficiency of 81m/W. Moreover, when the OEL device was driven with a constant current of 50mA/cm², the initial brightness was 2600cd/m², and the life property of the same was indicated by the brightness half-decay time of 450 hours.

### Example 11

An OEL device was obtained with the same method in Example 8, except that ALQ used as the material of the electron-transporting layer in Example 8 was replaced by PyPySPyPy. The ITO electrode served as an anode and the LiF/Al electrode as a cathode, while a DC voltage of about 3V was applied between them to produce a current of about 1mA/cm² and cause emission of blue light of 455nm in a luminescence efficiency of 8.51m/W. Moreover, when the OEL device was driven with a constant current of 50mA/cm², the initial brightness was 3800cd/m², and the life property of the same was indicated by the brightness half-decay time of 220 hours.

### Example 12

An OEL device was obtained with the same method in Example 11, except that the compound (32) used in Example 11 was replaced by the compound (40). The ITO electrode served as an anode and the LiF/Al electrode as a cathode, while a DC voltage of about 3.3V was applied between them to produce a current of about 1.2mA/cm² and cause emission of blue light of 454nm in a luminescence efficiency of 8.31m/W. Moreover, when the OEL device is driven with a constant current of 50mA/cm², the initial brightness was 3700cd/m², and the life property of the same was indicated by the brightness half-decay time of 200 hours.

### Comparative Example 1

An OEL device was obtained with the same method in Example 7, except that the compound (32) used in Example 7 was replaced by an anthracene derivative expressed by formula (106) below. The ITO electrode served as an anode and the LiF/Al electrode as a cathode, while a DC voltage of about 5V was applied between them to produce a current of about 3.5mA/cm² and cause emission of blue light of 467nm in a luminescence efficiency of 21m/W. Moreover, when the OEL device is driven with a constant current of 50mA/cm², the initial brightness was 1400cd/m², and the life property of the same was indicated by the brightness half-decay time of 125 hours.

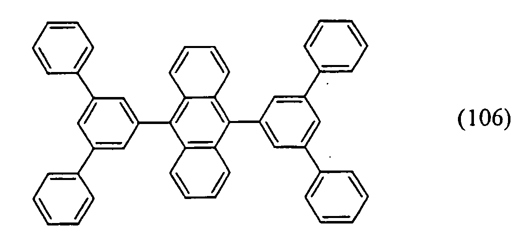

### Comparative Example 2

An OEL device was obtained with the same method in Example 10, except that the compound (32) used in Example 10 was replaced by an anthracene derivative expressed by formula (107) below. The ITO electrode served as an anode and the LiF/Al electrode as a cathode, while a DC voltage of about 4V was applied between them to produce a current of about 2mA/cm² and cause emission of blue light of 464nm in a luminescence efficiency of 41m/W. Moreover; when the OEL device is driven with a constant current of 50mA/cm², the initial brightness was 2400cd/m², and the life property of the same was indicated by the brightness half-decay time of 75 hours.

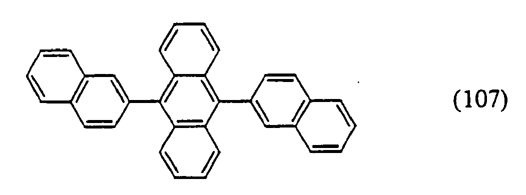

### Comparative Example 3

An OEL device was obtained with the same method in Example 4, except that the compound (32) used in Example 4 was replaced by the anthracene derivative (107) mentioned above. The ITO electrode served as an anode and the LiF/Al electrode as a cathode, while a DC voltage of about 6V was applied between them to produce a current of about 5mA/cm² and cause emission of blue light of 440nm in a luminescence efficiency of 1.21m/W. Moreover, when the OEL device is driven with a constant current of 50mA/cm², the initial brightness was 950cd/m², and the life property of the same was indicated by the brightness half-decay time of 50 hours.

## Claims

1. A luminescent material, expressed by formula (1): 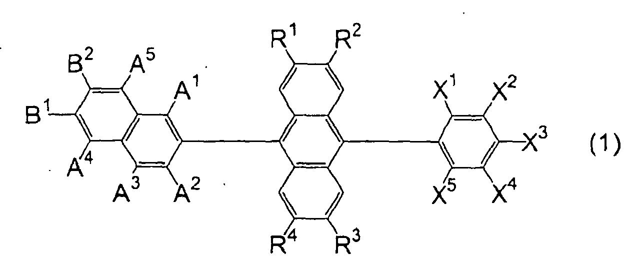 wherein
each of R¹-R⁴ represents hydrogen, a C₁-C₂₄ alkyl group or a C₁-C₂₄ alkoxy group;
each of A¹-A⁵ represents hydrogen, a C₁-C₂₄ alkyl group or a C₃-C₂₄ cycloalkyl group;
each of B¹ and B² represents hydrogen, or a C₆-C₂₄ aryl group, wherein B¹ and B² are not simultaneously hydrogen and any hydrogen on the C₆-C₂₄ aryl group can be substituted by a C₁-C₁₂ alkyl group, a C₃-C₁₂ cycloalkyl group or a C₆-C₁₂ aryl group;
each of X¹-X⁵ represents hydrogen, a C₁-C₂₄ alkyl group or a C₃-C₂₄ cycloalkyl group, wherein any methylene group of the C₁-C₂₄ alkyl group can be substituted by an oxy moiety; any methylene group of the C₁-C₂₄ alkyl group except the one directly bonded to the phenyl group can be substituted by a C₆-C₂₄ arylene group; and any hydrogen on the C₃-C₂₄ cycloalkyl group can be substituted by a C₁-C₂₄ alkyl group or a C₆-C₁₂ aryl group.

2. The luminescent material of claim 1, wherein
each of R¹-R⁴ represents hydrogen, methyl or *t*-butyl;
each of A¹-A⁵ represents hydrogen, methyl, *t*-butyl or cyclohexyl;
each of B¹ and B² represents hydrogen, or represents a phenyl, biphenylyl, terphenylyl, quaterphenylyl, naphthyl, phenanthryl, crycenyl or triphenylenyl group, on which any hydrogen can be substituted by a C₁-C₁₂ alkyl group, a C₃-C₁₂ cycloalkyl group or a C₆-C₁₂ aryl group; and
each of X¹-X⁵ represents hydrogen, a C₁-C₁₂ alkyl group or a C₃-C₁₂ cycloalkyl group.

3. The luminescent material of claim 1, wherein in the formula (1):
each of R¹-R⁴ represents hydrogen, methyl or *t*-butyl;
each of A¹-A⁵ represents hydrogen;
each of B¹ and B² represents hydrogen, phenyl, 2-biphenylyl, 3-biphenylyl, *m-*terphenyl-5'-yl, *m-*terphenyl-3-yl, 1-naphthyl, 2-naphthyl, 2-(2-naphthyl)phenyl, 3,5-di(1-naphthyl)phenyl, 3,5-di(2-naphthyl)phenyl, *p-*ter-phenyl-2'-yl, *m-*terphenyl-2-yl, *o-*terphenyl-2-yl, *p-*terphenyl-2-yl, 5'-phenyl-*m-*terphenyl-2-yl, 5'-phenyl-*m*-terphenyl-3-yl, *m-*quaterphenyl-2-yl, *m-*quaterphenyl-3-yl, 6-(*m-*terphenyl-5'-yl)-2-naphthyl or 4-(*m*-terphenyl-5'-yl)-1-naphthyl, wherein B¹ and B² are not simultaneously hydrogen; and
each of X¹-X⁵ represents hydrogen, methyl, *t*-butyl or cyclohexyl.

4. The luminescent material of claim 3, wherein each of X¹-X⁵ represents hydrogen.

5. The luminescent material of claim 3, wherein X³ represents *t*-butyl and each of X¹, X², X⁴ and X⁵ represents hydrogen.

6. The luminescent material of claim 3, wherein
among X¹, X³ and X⁵, at least one represents methyl and the rest represents hydrogen; and
each of X² and X⁴ represents hydrogen.

7. The luminescent material of claim 3, wherein each of B¹ and B² represents phenyl, each of X¹ and X⁵ represents hydrogen or methyl, each of X² and X⁴ represents hydrogen, and X³ represents hydrogen, *t*-butyl or methyl.

8. The luminescent material of claim 3, wherein B¹ represents phenyl, B² represents hydrogen, each of X¹ and X⁵ represents hydrogen or methyl, each of X² and X⁴ represents hydrogen, and X³ represents hydrogen, *t*-butyl or methyl.

9. The luminescent material of claim 3, wherein B¹ represents hydrogen, B² represents phenyl, each of X¹ and X⁵ represents hydrogen or methyl, each of X² and X⁴ represents hydrogen, and X³ represents hydrogen, *t*-butyl or methyl.

10. The luminescent material of claim 3, wherein each of B¹ and B² represents 2-biphenylyl, each of X¹ and X⁵ represents hydrogen or methyl, each of X² and X⁴ represents hydrogen, and X³ represents hydrogen, *t*-butyl or methyl.

11. The luminescent material of claim 3, wherein B¹ represents 2-biphenylyl, B² represents hydrogen, each of X¹ and X⁵ represents hydrogen or methyl, each of X² and X⁴ represents hydrogen, and X³ represents hydrogen, *t*-butyl or methyl.

12. The luminescent material of claim 3, wherein B¹ represents hydrogen, B² represents 2-biphenylyl, each of X¹ and X⁵ represents hydrogen or methyl, each of X² and X⁴ represents hydrogen, and X³ represents hydrogen, *t*-butyl or methyl.

13. The luminescent material of claim 3, wherein each of B¹ and B² represents 3-biphenylyl, each of X¹ and X⁵ represents hydrogen or methyl, each of X² and X⁴ represents hydrogen, and X³ represents hydrogen, *t*-butyl or methyl.

14. The luminescent material of claim 3, wherein B¹ represents 3-biphenylyl, B² represents hydrogen, each of X¹ and X⁵ represents hydrogen or methyl, each of X² and X⁴ represents hydrogen, and X³ represents hydrogen, *t*-butyl or methyl.

15. The luminescent material of claim 3, wherein B¹ represents hydrogen, B² represents 3-biphenylyl, each of X¹ and X⁵ represents hydrogen or methyl, each of X² and X⁴ represents hydrogen, and X³ represents hydrogen, *t*-butyl or methyl.

16. The luminescent material of claim 3, wherein B¹ represents *m-*terphenyl-5'-yl, B² represents hydrogen, each of X¹ and X⁵ represents hydrogen or methyl, each of X² and X⁴ represents hydrogen, and X³ represents hydrogen, *t*-butyl or methyl.

17. The luminescent material of claim 3, wherein B¹ represents hydrogen, B² represents *m-*terphenyl-5'-yl, each of X¹ and X⁵ represents hydrogen or methyl, each of X² and X⁴ represents hydrogen, and X³ represents hydrogen, *t*-butyl or methyl.

18. The luminescent material of claim 3, wherein B¹ represents *m-*terphenyl-3-yl, B² represents hydrogen, each ofX' and X⁵ represents hydrogen or methyl, each of X² and X⁴ represents hydrogen, and X³ represents hydrogen, *t*-butyl or methyl.

19. The luminescent material of claim 3, wherein B¹ represents hydrogen, B² represents *m-*terphenyl-3-yl, each of X¹ and X⁵ represents hydrogen or methyl, each of X² and X⁴ represents hydrogen, and X³ represents hydrogen, *t*-butyl or methyl.

20. The luminescent material of claim 3, wherein B¹ represents 1-naphthyl, B² represents hydrogen, each of X¹ and X⁵ represents hydrogen or methyl, each of X² and X⁴ represents hydrogen, and X³ represents hydrogen, *t*-butyl or methyl.

21. The luminescent material of claim 3, wherein B¹ represents hydrogen, B² represents 1-naphthyl, each of X¹ and X⁵ represents hydrogen or methyl, each of X² and X⁴ represents hydrogen, and X³ represents hydrogen, *t*-butyl or methyl.

22. The luminescent material of claim 3, wherein B¹ represents 2-naphthyl, B² represents hydrogen, each of X¹ and X⁵ represents hydrogen or methyl, each of X² and X⁴ represents hydrogen, and X³ represents hydrogen, *t*-butyl or methyl.

23. The luminescent material of claim 3, wherein B¹ represents hydrogen, B² represents 2-naphthyl, each of X¹ and X⁵ represents hydrogen or methyl, each of X² and X⁴ represents hydrogen, and X³ represents hydrogen, *t*-butyl or methyl.

24. The luminescent material of claim 3, wherein B¹ represents 2-(2-naphthyl)phenyl, B² represents hydrogen, each of X¹ and X⁵ represents hydrogen or methyl, each of X² and X⁴ represents hydrogen, and X³ represents hydrogen, *t*-butyl or methyl.

25. The luminescent material of claim 3, wherein B¹ represents hydrogen, B² represents 2-(2-naphthyl)phenyl, each of X¹ and X⁵ represents hydrogen or methyl, each of X² and X⁴ represents hydrogen, and X³ represents hydrogen, *t*-butyl or methyl.

26. The luminescent material of claim 3, wherein B¹ represents 3,5-di(1-naphthyl)phenyl or 3,5-di(2-naphthyl)phenyl, B² represents hydrogen, each of
X¹ and X⁵ represents hydrogen or methyl, each of X² and X⁴ represents hydrogen, and X³ represents hydrogen, *t*-butyl or methyl.

27. The luminescent material of claim 3, wherein B¹ represents hydrogen, B² represents 3,5-di(1-naphthyl)phenyl or 3,5-di(2-naphthyl)phenyl, each of X¹ and X⁵ represents hydrogen or methyl, each of X² and X⁴ represents hydrogen, and X³ represents hydrogen, *t*-butyl or methyl.

28. The luminescent material of claim 3, wherein B¹ represents a group selected from *p-*terphenyl-2'-yl, *m-*terphenyl-2-yl, *o*-terphenyl-2-yl and *p-*terphenyl-2-yl, B² represents hydrogen, each of X¹ and X⁵ represents hydrogen or methyl, each of X² and X⁴ represents hydrogen, and X³ represents hydrogen, *t*-butyl or methyl.

29. The luminescent material of claim 3, wherein B¹ represents hydrogen, B² represents a group selected from *p*-terphenyl-2'-yl, *m-*terphenyl-2-yl, *o-*terphenyl-2-yl and *p-*terphenyl-2-yl, each of X¹ and X⁵ represents hydrogen or methyl, each of X² and X⁴ represents hydrogen, and X³ represents hydrogen, *t*-butyl or methyl.

30. The luminescent material of claim 3, wherein B¹ represents 5'-phenyl-*m-*terphenyl-2-yl or 5'-phenyl-*m-*terphenyl-3-yl, B² represents hydrogen, each of X¹ and X⁵ represents hydrogen or methyl, each of X² and X⁴ represents hydrogen, and X³ represents hydrogen, *t*-butyl or methyl.

31. The luminescent material of claim 3, wherein B¹ represents hydrogen, B² represents 5'-phenyl-*m-*terphenyl-2-yl or 5'-phenyl-*m-*terphenyl-3-yl, each of X¹ and X⁵ represents hydrogen or methyl, each of X² and X⁴ represents hydrogen, and X³ represents hydrogen, *t*-butyl or methyl.

32. The luminescent material of claim 3, wherein B¹ represents *m-*quaterphenyl-2-yl or *m-*quaterphenyl-3-yl, B² represents hydrogen, each of X¹ and X⁵ represents hydrogen or methyl, each of X² and X⁴ represents hydrogen, and X³ represents hydrogen, *t*-butyl or methyl.

33. The luminescent material of claim 3, wherein B¹ represents hydrogen, B² represents *m-*quaterphenyl-2-yl or *m*-quaterphenyl-3-yl, each of X¹ and X⁵ represents hydrogen or methyl, each of X² and X⁴ represents hydrogen, and X³ represents hydrogen, *t*-butyl or methyl.

34. The luminescent material of claim 3, wherein B¹ represents 6-(*m-*terphenyl-5'-yl)-2-naphthyl or 4-(*m-*terphenyl-5'-yl)-1-naphthyl, B² represents hydrogen, each of X¹ and X⁵ represents hydrogen or methyl, each of X² and X⁴ represents hydrogen, and X³ represents hydrogen, *t*-butyl or methyl.

35. The luminescent material of claim 3, wherein B¹ represents hydrogen, B² represents 6-(*m-*terphenyl-5'-yl)-2-naphthyl or 4-(*m-*terphenyl-5'-yl)-1-naphthyl, each of X¹ and X⁵ represents hydrogen or methyl, each of X² and X⁴ represents hydrogen, and X³ represents hydrogen, *t*-butyl or methyl.

36. An organic electroluminescent device, comprising at least a hole-transporting layer, a luminescent layer and an electron-transporting layer that are sandwiched between an anode and a cathode on a substrate, wherein the luminescent layer contains the luminescent material of claim 1.

37. The organic electroluminescent device of claim 36, wherein the luminescent layer contains at least one luminescent dopant selected from perylene derivatives, borane derivatives, amine-containing styryl derivatives, aromatic amine derivatives, coumarin derivatives, pyran derivatives, iridium complexes and platinum complexes.

38. The organic electroluminescent device of claim 36, wherein the luminescent layer contains a perylene derivative as a luminescent dopant.

39. The organic electroluminescent device of claim 36, wherein the luminescent layer contains a borane derivative as a luminescent dopant.

40. The organic electroluminescent device of claim 36, wherein the luminescent layer contains an amine-containing styryl derivative as a luminescent dopant.

41. The organic electroluminescent device of claim 36, wherein the luminescent layer contains an aromatic amine derivative as a luminescent dopant.

42. The organic electroluminescent device of claim 36, wherein the luminescent layer contains a coumarin derivative as a luminescent dopant.

43. The organic electroluminescent device of claim 36, wherein the luminescent layer contains a pyran derivative as a luminescent dopant.

44. The organic electroluminescent device of claim 36, wherein the luminescent layer contains an iridium complex as a luminescent dopant.

45. The organic electroluminescent device of claim 36, wherein the luminescent layer contains a platinum complex as a luminescent dopant.

46. The organic electroluminescent device of claim 36, wherein the electron-transporting layer comprises a quinolinol metal complex.

47. The organic electroluminescent device of claim 36, wherein the electron-transporting layer comprises at least one of a pyridine derivative and a phenanthroline derivative.

## Patentansprüche

1. Lumineszenzmaterial, dargestellt durch die Formel (1): 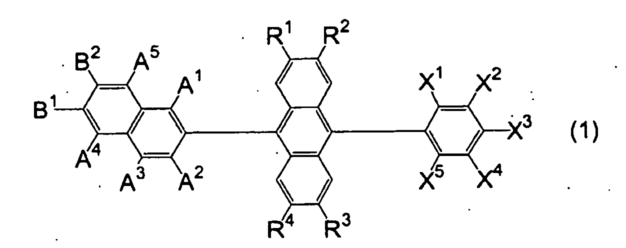 worin
jedes von R¹ bis R⁴ Wasserstoff, eine C₁-C₂₄-Alkylgruppe oder eine C₁-C₂₄-Alkoxygruppe ist;
jedes von A¹ bis A⁵ Wasserstoff, eine C₁-C₂₄-Alkylgruppe oder eine C₃-C₂₄-Cycloalkylgruppe ist;
jedes von B¹ und B² Wasserstoff oder eine C₆-C₂₄-Arylgruppe ist, worin B¹ und B² nicht gleichzeitig Wasserstoff sind, und irgendein Wasserstoff an der C₆-C₂₄-Arylgruppe durch eine C₁-C₁₂-Alkylgruppe, C₃-C₂₄-Cycloalkylgruppe oder C₆-C₂₄-Arylgruppe substituiert sein kann; und
jedes von X¹ bis X⁵ Wasserstoff, eine C₁-C₂₄-Alkylgruppe oder eine C₃-C₂₄-Cycloalkylgruppe ist, worin irgendeine Methylengruppe der C₁-C₂₄-Alkylgruppe durch einen Oxyanteil substituiert sein kann; irgendeine Methylengruppe der C₁-C₂₄-Alkylgruppe, mit Ausnahme der, die direkt an die Phenylgruppe gebunden ist, durch eine C₆-C₂₄-Arylengruppe substituiert sein kann; und irgendein Wasserstoff an der C₃-C₂₄-Cycloalkylgruppe durch eine C₁-C₂₄-Alkylgruppe oder C₆-C₁₂-Arylgruppe substituiert sein kann.

2. Lumineszenzmaterial nach Anspruch 1, worin R¹ bis R⁴ jeweils Wasserstoff, Methyl oder t-Butyl sind;
A¹ bis A⁵ jeweils Wasserstoff, Methyl, t-Butyl oder Cyclohexyl sind;
B¹ und B² jeweils Wasserstoff sind oder eine Phenyl-, Biphenyl-, Terphenylyl-, Quaterphenylyl-, Naphthyl-, Phenanthryl-, Crycenyl- oder Triphenylenyl-Gruppe sind, wobei irgendein Wasserstoff durch eine C₁-C₁₂-Alkylgruppe, C₃-C₁₂-Cycloalkylgruppe oder C₆-C₁₂-Arylgruppe substituiert sein kann; und
X¹ bis X⁵ jeweils Wasserstoff, eine C₁-C₁₂-Alkylgruppe oder eine C₃-C₁₂-Cycloalkylgruppe sind.

3. Lumineszenzmaterial nach Anspruch 1, worin in der Formel (1) R¹ bis R⁴ jeweils Wasserstoff, Methyl oder t-Butyl sind;
A¹ bis A⁵ jeweils Wasserstoff sind;
B¹ und B² jeweils Wasserstoff, Phenyl, 2-Biphenylyl, 3-Biphenylyl, m-Terphenyl-5'-yl, m-Terphenyl-3-yl, 1-Naphthyl, 2-Naphthyl, 2-(2-Naphthyl)phenyl, 3,5-Di(1-naphthyl)phenyl, 3,5-Di(2-naphthyl)phenyl, p-Terphenyl-2'-yl, m-Terphenyl-2-yl, o-Terphenyl-2-yl, p-Terphenyl-2-yl, 5'-Phenyl-m-terphenyl-2-yl, 5'-Phenyl-m-terphenyl-3-yl, m-Quaterphenyl-2-yl, m-Quaterphenyl-3-yl, 6-(m-Terphenyl-5'-yl)-2-naphthyl oder 4-(m-Terphenyl-5'-yl)-1-naphthyl sind; worin B¹ und B² nicht gleichzeitig Wasserstoff sind; und
X¹ bis X⁵ jeweils Wasserstoff, Methyl, t-Butyl oder Cyclohexyl sind.

4. Lumineszenzmaterial nach Anspruch 3, worin X¹ bis X⁵ jeweils Wasserstoff sind.

5. Lumineszenzmaterial nach Anspruch 3, worin X³ t-Butyl ist und X¹ X², X⁴ und X⁵ jeweils Wasserstoff sind.

6. Lumineszenzmaterial nach Anspruch 3, worin von X¹, X³ und X⁵ zumindest eines Methyl und der Rest Wasserstoff sind; und
X² und X⁴ jeweils Wasserstoff sind.

7. Lumineszenzmaterial nach Anspruch 3, worin B¹ und B² jeweils Phenyl sind, X¹ und X⁵ jeweils Wasserstoff oder Methyl sind, X² und X⁴ jeweils Wasserstoff sind und X³ Wasserstoff, t-Butyl oder Methyl ist.

8. Lumineszenzmaterial nach Anspruch 3, worin B¹ Phenyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

9. Lumineszenzmaterial nach Anspruch 3, worin B¹ Wasserstoff ist, B² Phenyl ist, X¹ und X⁵ jeweils Wasserstoff oder Methyl sind, X² und X⁴ jeweils Wasserstoff sind und X³ Wasserstoff, t-Butyl oder Methyl ist.

10. Lumineszenzmaterial nach Anspruch 3, worin B¹ und B² jeweils 2-Biphenylyl sind, X¹ und X⁵ jeweils Wasserstoff oder Methyl sind, X² und X⁴ jeweils Wasserstoff sind und X³ Wasserstoff, t-Butyl oder Methyl ist.

11. Lumineszenzmaterial nach Anspruch 3, worin B¹ 2-Biphenylyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

12. Lumineszenzmaterial nach Anspruch 3, worin B¹ Wasserstoff ist, B² 2-Biphenylyl ist, X¹ und X⁵ jeweils Wasserstoff oder Methyl sind, X² und X⁴ jeweils Wasserstoff sind und X³ Wasserstoff, t-Butyl oder Methyl ist.

13. Lumineszenzmaterial nach Anspruch 3, worin B¹ und B² jeweils 3-Biphenylyl sind, X¹ und X⁵ jeweils Wasserstoff oder Methyl sind, X² und X⁴ jeweils Wasserstoff sind und X³ Wasserstoff, t-Butyl oder Methyl ist.

14. Lumineszenzmaterial nach Anspruch 3, worin B¹ 3-Biphenylyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

15. Lumineszenzmaterial nach Anspruch 3, worin B¹ Wasserstoff ist, B² 3-Biphenylyl ist, X¹ und X⁵ jeweils Wasserstoff oder Methyl sind, X² und X⁴ jeweils Wasserstoff sind und X³ Wasserstoff, t-Butyl oder Methyl ist.

16. Lumineszenzmaterial nach Anspruch 3, worin B¹ m-Terphenyl-5'-yl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

17. Lumineszenzmaterial nach Anspruch 3, worin B¹ Wasserstoff ist, B² m-Terphenyl-5'-yl ist, X¹ und X⁵ jeweils Wasserstoff oder Methyl sind, X² und X⁴ jeweils Wasserstoff sind und X³ Wasserstoff, t-Butyl oder Methyl ist.

18. Lumineszenzmaterial nach Anspruch 3, worin B¹ m-Terphenyl-5'-yl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

19. Lumineszenzmaterial nach Anspruch 3, worin B¹ Wasserstoff ist, B² m-Terphenyl-3-yl sind, X¹ und X⁵ jeweils Wasserstoff oder Methyl sind, X² und X⁴ jeweils Wasserstoff sind und X³ Wasserstoff, t-Butyl oder Methyl ist.

20. Lumineszenzmaterial nach Anspruch 3, worin B¹ 1-Naphthyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

21. Lumineszenzmaterial nach Anspruch 3, worin B¹ Wasserstoff ist, B² 1-Naphthyl ist, X¹ und X⁵ jeweils Wasserstoff oder Methyl sind, X² und X⁴ jeweils Wasserstoff sind und X³ Wasserstoff, t-Butyl oder Methyl ist.

22. Lumineszenzmaterial nach Anspruch 3, worin B¹ 2-Naphthyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

23. Lumineszenzmaterial nach Anspruch 3, worin B¹ Wasserstoff ist, B² 2-Naphthyl ist, X¹ und X⁵ jeweils Wasserstoff oder Methyl sind, X² und X⁴ jeweils Wasserstoff sind und X³ Wasserstoff, t-Butyl oder Methyl ist.

24. Lumineszenzmaterial nach Anspruch 3, worin B¹ 2-(2-Naphthyl)phenyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

25. Lumineszenzmaterial nach Anspruch 3, worin B¹ Wasserstoff ist, B² 2-(2-Naphthyl)phenyl ist, X¹ und X⁵ jeweils Wasserstoff oder Methyl sind, X² und X⁴ jeweils Wasserstoff sind und X³ Wasserstoff, t-Butyl oder Methyl ist.

26. Lumineszenzmaterial nach Anspruch 3, worin B¹ 3,5-Di(1-naphthyl)phenyl oder 3,5-Di(2-naphthyl)phenyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

27. Lumineszenzmaterial nach Anspruch 3, worin B¹ Wasserstoff ist, B² 3,5-Di(1-naphthyl)phenyl oder 3,5-Di(2-naphthyl)phenyl ist, X¹ und X⁵ jeweils Wasserstoff oder Methyl sind, X² und X⁴ jeweils Wasserstoff sind und X³ Wasserstoff, t-Butyl oder Methyl ist.

28. Lumineszenzmaterial nach Anspruch 3, worin B¹ eine Gruppe ist, bestehend aus p-Terphenyl-2'-yl, m-Terphenyl-2-yl, o-Terphenyl-2-yl und p-Terphenyl-2-yl, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

29. Lumineszenzmaterial nach Anspruch 3, worin B¹ Wasserstoff ist, B² eine Gruppe ist, ausgewählt aus p-Terphenyl-2'-yl, m-Terphenyl-2-yl, o-Terphenyl-2-yl und p-Terphenyl-2-yl, X¹ und X⁵ jeweils Wasserstoff oder Methyl sind, X² und X⁴ jeweils Wasserstoff sind und X³ Wasserstoff, t-Butyl oder Methyl ist.

30. Lumineszenzmaterial nach Anspruch 3, worin B¹ 5'-Phenyl-m-terphenyl-2-yl oder 5'-Phenyl-m-terphenyl-3-yl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

31. Lumineszenzmaterial nach Anspruch 3, worin B¹ Wasserstoff ist, B² 5'-Phenyl-m-terphenyl-2-yl oder 5'-Phenyl-m-terphenyl-3-yl ist, X¹ und X⁵ jeweils Wasserstoff oder Methyl sind, X² und X⁴ jeweils Wasserstoff sind und X³ Wasserstoff, t-Butyl oder Methyl ist.

32. Lumineszenzmaterial nach Anspruch 3, worin B¹ m-Quaterphenyl-2-yl oder m-Quaterphenyl-3-yl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

33. Lumineszenzmaterial nach Anspruch 3, worin B¹ Wasserstoff ist, B² m-Quaterphenyl-2-yl oder m-Quaterphenyl-3-yl ist, X¹ und X⁵ jeweils Wasserstoff oder Methyl sind, X² und X⁴ jeweils Wasserstoff sind und X³ Wasserstoff, t-Butyl oder Methyl ist.

34. Lumineszenzmaterial nach Anspruch 3, worin B¹ 6-(m-Terphenyl-5'-yl)-2-naphthyl oder 4-(m-Terphenyl-5'-yl)-1-naphthyl ist, B² Wasserstoff ist, X¹ und X⁵ jeweils Wasserstoff oder Methyl sind, X² und X⁴ jeweils Wasserstoff sind und X³ Wasserstoff, t-Butyl oder Methyl ist.

35. Lumineszenzmaterial nach Anspruch 3, worin B¹ Wasserstoff ist, B² 6-(m-Terphenyl-5'-yl)-2-naphthyl oder 4-(m-Terphenyl-5'-yl)-1-naphthyl ist, X¹ und X⁵ jeweils Wasserstoff oder Methyl sind, X² und X⁴ jeweils Wasserstoff sind und X³ Wasserstoff, t-Butyl oder Methyl ist.

36. Organische Elektrolumineszenzvorrichtung, umfassend eine Loch-Transportschicht, eine Lumineszenzschicht und eine Elektronen-Transportschicht, die zwischen einer Anode und einer Kathode auf einem Substrat in Sandwichform angeordnet sind, worin die Lumineszenzschicht ein Lumineszenzmaterial nach Anspruch 1 umfasst.

37. Organische Elektrolumineszenzvorrichtung nach Anspruch 36, worin die Lumineszenzschicht zumindest ein Lumineszenz-Dotiermittel umfasst, ausgewählt aus Perylenderivaten, Boranderivaten, Amin-haltigen Styrylderivaten, aromatischen Aminderivaten, Kumarinderivaten, Pyranderivaten, Iridiumkomplexen und Platinkomplexen.

38. Organische Elektrolumineszenzvorrichtung nach Anspruch 36, worin die Lumineszenzschicht ein Perylenderivat als Lumineszenz-Dotiermittel umfasst.

39. Organische Elektrolumineszenzvorrichtung nach Anspruch 36, worin die Lumineszenzschicht ein Boranderivat als Lumineszenz-Dotiermittel umfasst.

40. Organische Elektrolumineszenzvorrichtung nach Anspruch 36, worin die Lumineszenzschicht ein Amin-haltiges Styrylderivat als Lumineszenz-Dotiermittel umfasst.

41. Organische Elektrolumineszenzvorrichtung nach Anspruch 36, worin die Lumineszenzschicht ein aromatisches Aminderivat als Lumineszenz-Dotiermittel umfasst.

42. Organische Elektrolumineszenzvorrichtung nach Anspruch 36, worin die Lumineszenzschicht ein Kumarinderivat als Lumineszenz-Dotiermittel umfasst.

43. Organische Elektrolumineszenzvorrichtung nach Anspruch 36, worin die Lumineszenzschicht ein Pyranderivat als Lumineszenz-Dotiermittel umfasst.

44. Organische Elektrolumineszenzvorrichtung nach Anspruch 36, worin die Lumineszenzschicht einen Iridiumkomplex als Lumineszenz-Dotiermittel umfasst.

45. Organische Elektrolumineszenzvorrichtung nach Anspruch 36, worin die Lumineszenzschicht einen Platinkomplex als Lumineszenz-Dotiermittel umfasst.

46. Organische Elektrolumineszenzvorrichtung nach Anspruch 36, worin die Elektronen-Transportschicht einen Chinolinolmetallkomplex umfasst.

47. Organische Elektrolumineszenzvorrichtung nach Anspruch 36, worin die Elektronen-Transportschicht zumindest eines von einem Pyridinderivat und einem Phenanthrolinderivat umfasst.

## Revendications

1. Matière luminescente, exprimée par la formule (1) : 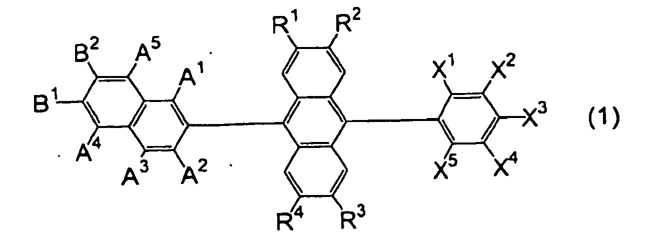 dans laquelle
chacun de R¹ - R⁴ représente de l'hydrogène, un groupe alkyle C₁ - C₂₄ ou un groupe alcoxy C₁ - C₂₄ ;
chacun de A¹ - A⁵ représente de l'hydrogène, un groupe alkyle C₁ - C₂₄ ou un groupe cycloalkyle C₃ - C₂₄ ;
chacun de B¹ et B² représente de l'hydrogène, ou un groupe aryle C₆ - C₂₄, dans lesquels B¹ et B² ne sont pas simultanément de l'hydrogène et n'importe quel hydrogène sur le groupe aryle C₆ - C₂₄ peut être substitué par un groupe alkyle C₁ - C₁₂, un groupe cycloalkyle C₃ - C₁₂ ou un groupe aryle C₆ - C₁₂ ;
chacun de X¹ - X⁵ représente de l'hydrogène, un groupe alkyle C₁ - C₂₄ ou un groupe cycloalkyle C₃ - C₂₄, dans lequel n'importe quel groupe méthylène du groupe alkyle C₁ - C₂₉ peut être substitué par un motif oxy ; n'importe quel groupe méthylène du groupe alkyle C₁ - C₂₄ sauf celui directement lié au groupe phényle peut être substitué par un groupe arylène C₆ - C₂₄ ; et n'importe quel hydrogène sur le groupe cycloalkyle C₃ - C₂₄ peut être substitué par un groupe alkyle C₁ - C₂₄ ou un groupe aryle C₆ - C₁₂.

2. Matière luminescente selon la revendication 1, dans laquelle
chacun de R¹ - R⁴ représente de l'hydrogène, du méthyle ou du *t*-butyle ;
chacun de A¹ - A⁵ représente de l'hydrogène, du méthyle, du *t*-butyle ou du cyclohexyle ;
chacun de B¹ et B² représente de l'hydrogène ou représente un groupe phényle, biphénylyle, terphénylyle, quaterphénylyle, naphthyle, phénanthryle, crycényle ou triphénylènyle, sur lequel n'importe quel hydrogène peut être substitué par un groupe alkyle C₁ - C₁₂, un groupe cycloalkyle C₃ - C₁₂ ou un groupe aryle C₆ - C₁₂ ; et
chacun de X¹ - X⁵ représente de l'hydrogène, un groupe alkyle C₁ - C₁₂ ou un groupe cycloalkyle C₃ - C₁₂.

3. Matière luminescente selon la revendication 1, dans laquelle dans la formule (1) :
chacun de R¹ - R⁴ représente de l'hydrogène, du méthyle ou du *t*-butyle ;
chacun de A¹ - A⁵ représente de l'hydrogène ;
chacun de B¹ et B² représente de l'hydrogène, du phényle, du 2-biphénylyle, du 3-biphénylyle, du *m-*terphényl-5'-yle, du *m-*terphényl-3-yle, du 1-naphthyle, du 2-naphthyle, du 2-(2-naphthyl)phényle, du 3,5-di(1-naphthyl)phényle, du 3,5-di(2-naphthyle)phényle, du *p-*terphényl-2'-yle, du *m-*terphényl-2-yle, du *o-*terphényl-2-yle, du *p-*terphényl-2-yle, du 5'-phényl-*m-*terphényl-2-yle, du 5'-phényl-*m-*terphényl-3-yle, du *m-*quaterphényl-2-yle, du *m-*quaterphényl-3-yle, du 6-(*m-*terphényl-5'-yl)-2-naphthyle ou du 4-(*m-*terphényl-5'-yl)-1-naphthyle,
dans laquelle B¹ et B² ne sont pas simultanément de l'hydrogène ; et
chacun de X¹ - X⁵ représente de l'hydrogène, du méthyle, du *t*-butyle ou du cyclohexyle.

4. Matière luminescente selon la revendication 3, dans laquelle chacun de X¹ - X⁵ représente de l'hydrogène.

5. Matière luminescente selon la revendication 3, dans laquelle X³ représente du *t*-butyle et chacun de X¹, X², X⁴ et X⁵ représente de l'hydrogène.

6. Matière luminescente selon la revendication 3, dans laquelle
parmi X¹, X³ et X⁵, au moins un représente du méthyle et le reste représente de l'hydrogène ; et
chacun de X² et X⁴ représente de l'hydrogène.

7. Matière luminescente selon la revendication 3, dans laquelle chacun de B¹ et B² représente du phényle, chacun de X¹ et X⁵ représente de l'hydrogène ou du méthyle, chacun de X² et x⁴ représente de l'hydrogène, et X³ représente de l'hydrogène, du *t-*butyle ou du méthyle.

8. Matière luminescente selon la revendication 3, dans laquelle B¹ représente du phényle, B² représente de l'hydrogène, chacun de X¹ et X⁵ représente de l'hydrogène ou du méthyle, chacun de X² et X⁴ représente de l'hydrogène, et X³ représente de l'hydrogène, du *t-*butyle ou du méthyle.

9. Matière luminescente selon la revendication 3, dans laquelle B¹ représente de l'hydrogène, B² représente du phényle, chacun de X¹ et X⁵ représente de l'hydrogène ou du méthyle, chacun de X² et X⁴ représente de l'hydrogène, et X³ représente de l'hydrogène, du *t-*butyle ou du méthyle.

10. Matière luminescente selon la revendication 3, dans laquelle chacun de B¹ et B² représente du 2-biphénylyle, chacun de X¹ et X⁵ représente de l'hydrogène ou du méthyle, chacun de X² et X⁴ représente de l'hydrogène, et X³ représente de l'hydrogène, du *t-*butyle ou du méthyle.

11. Matière luminescente selon la revendication 3, dans laquelle B¹ représente du 2-biphénylyle, B² représente de l'hydrogène, chacun de X¹ et X⁵ représente de l'hydrogène ou du méthyle, chacun de X² et X⁴ représente de l'hydrogène, et X³ représente de l'hydrogène, du *t*-butyle ou du méthyle.

12. Matière luminescente selon la revendication 3, dans laquelle B¹ représente de l'hydrogène, B² représente du 2-biphénylyle, chacun de X¹ et X⁵ représente de l'hydrogène ou du méthyle, chacun de X² et X⁴ représente de l'hydrogène, et X³ représente de l'hydrogène, du *t*-butyle ou du méthyle.

13. Matière luminescente selon la revendication 3, dans laquelle chacun de B¹ et B² représente du 3-biphénylyle, chacun de X¹ et X⁵ représente de l'hydrogène ou du méthyle, chacun de X² et X⁴ représente de l'hydrogène, et X³ représente de l'hydrogène, du *t-*butyle ou du méthyle.

14. Matière luminescente selon la revendication 3, dans laquelle B¹ représente du 3-biphénylyle, B² représente de l'hydrogène, chacun de X¹ et X⁵ représente de l'hydrogène ou du méthyle, chacun de X² et X⁴ représente de l'hydrogène, et X3 représente de l'hydrogène, du *t*-butyle ou du méthyle.

15. Matière luminescente selon la revendication 3, dans laquelle B¹ représente de l'hydrogène, B² représente du 3-biphénylyle, chacun de X¹ et X⁵ représente de l'hydrogène ou du méthyle, chacun de X² et X⁴ représente de l'hydrogène, et X³ représente de l'hydrogène, du *t*-butyle ou du méthyle.

16. Matière luminescente selon la revendication 3, dans laquelle B¹ représente du *m-*terphényl-5'-yle, B² représente de l'hydrogène, chacun de X¹ et X⁵ représente de l'hydrogène ou du méthyle, chacun de X² et X⁴ représente de l'hydrogène, et X³ représente de l'hydrogène, du *t*-butyle ou du méthyle.

17. Matière luminescente selon la revendication 3, dans laquelle B¹ représente de l'hydrogène, B² représente du *m-*terphényl-5'-yle, chacun de X¹ et X⁵ représente de l'hydrogène ou du méthyle, chacun de X² et X⁴ représente de l'hydrogène, et X³ représente de l'hydrogène, du t-butyle ou du méthyle.

18. Matière luminescente selon la revendication 3, dans laquelle B¹ représente du *m-*terphényl-3-yle, B² représente de l'hydrogène, chacun de X¹ et X⁵ représente de l'hydrogène ou du méthyle, chacun de X² et X⁴ représente de l'hydrogène, et X³ représente de l'hydrogène, du *t*-butyle ou du méthyle.

19. Matière luminescente selon la revendication 3, dans laquelle B¹ représente de l'hydrogène, B² représente du m-terphényl-3-yle, chacun de X¹ et X⁵ représente de l'hydrogène ou du méthyle, chacun de X² et X⁴ représente de l'hydrogène, et X³ représente de l'hydrogène, du *t*-butyle ou du méthyle.

20. Matière luminescente selon la revendication 3, dans laquelle B¹ représente du 1-naphthyle, B² représente de l'hydrogène, chacun de X¹ et X⁵ représente de l'hydrogène ou du méthyle, chacun de X² et X⁴ représente de l'hydrogène, et X³ représente de l'hydrogène, du *t*-butyle ou du méthyle.

21. Matière luminescente selon la revendication 3, dans laquelle B¹ représente de l'hydrogène, B² représente du 1-naphthyle, chacun de X¹ et X⁵ représente de l'hydrogène ou du méthyle, chacun de X² et X⁴ représente de l'hydrogène, et X³ représente de l'hydrogène, du *t*-butyle ou du méthyle.

22. Matière luminescente selon la revendication 3, dans laquelle B¹ représente du 2-naphthyle, B² représente de l'hydrogène, chacun de X¹ et X⁵ représente de l'hydrogène ou du méthyle, chacun de X² et X⁴ représente de l'hydrogène, et X³ représente de l'hydrogène, du *t*-butyle ou du méthyle.

23. Matière luminescente selon la revendication 3, dans laquelle B¹ représente de l'hydrogène, B² représente du 2-naphthyle, chacun de X¹ et X⁵ représente de l'hydrogène ou du méthyle, chacun de X² et X⁴ représente de l'hydrogène, et X³ représente de l'hydrogène, du t-butyle ou du méthyle.

24. Matière luminescente selon la revendication 3, dans laquelle B¹ représente du 2-(2-naphthyl)phényle, B² représente de l'hydrogène, chacun de X¹ et X⁵ représente de l'hydrogène ou du méthyle, chacun de X² et X⁴ représente de l'hydrogène, et X³ représente de l'hydrogène, du t-butyle ou du méthyle.

25. Matière luminescente selon la revendication 3, dans laquelle B¹ représente de l'hydrogène, B² représente du 2-(2-naphthyl)phényle, chacun de X¹ et X⁵ représente de l'hydrogène ou du méthyle, chacun de X² et X⁴ représente de l'hydrogène, et X³ représente de l'hydrogène, du t-butyle ou du méthyle.

26. Matière luminescente selon la revendication 3, dans laquelle B¹ représente du 3,5-di(1-naphthyl)phényle ou du 3,5-di(2-naphthyl)phényle, B² représente de l'hydrogène, chacun de X¹ et X⁵ représente de l'hydrogène ou du méthyle, chacun de X² et X⁴ représente de l'hydrogène, et X³ représente de l'hydrogène, du t-butyle ou du méthyle.

27. Matière luminescente selon la revendication 3, dans laquelle B¹ représente de l'hydrogène, B² représente du 3,5-di(1-naphthyl)phényle ou du 3,5-di(2-naphthyl)phényle, chacun de X¹ et X⁵ représente de l'hydrogène ou du méthyle, chacun de X² et X⁴ représente de l'hydrogène, et X³ représente de l'hydrogène, du t-butyle ou du méthyle.

28. Matière luminescente selon la revendication 3, dans laquelle B¹ représente un groupe sélectionné parmi p-terphényl-2'-yle, m-terphényl-2-yle, o-terphényl-2-yle et p-terphényl-2-yle, B² représente de l'hydrogène, chacun de X¹ et X⁵ représente de l'hydrogène ou du méthyle, chacun de X² et X⁴ représente de l'hydrogène, et X³ représente de l'hydrogène, du t-butyle ou du méthyle.

29. Matière luminescente selon la revendication 3, dans laquelle B¹ représente de l'hydrogène, B² représente un groupe sélectionné parmi p-terphényl-2'-yle, m-terphényl-2-yle, o-terphényl-2-yle et p-terphényl-2-yle, chacun de X¹ et X⁵ représente de l'hydrogène ou du méthyle, chacun de X² et X⁴ représente de l'hydrogène, et X³ représente de l'hydrogène, du t-butyle ou du méthyle.

30. Matière luminescente selon la revendication 3, dans laquelle B¹ représente du 5'-phényl-m-terphényl-2-yle ou du 5'-phényl-m-terphényl-3-yle, B² représente de l'hydrogène, chacun de X¹ et X⁵ représente de l'hydrogène ou du méthyle, chacun de X² et X⁴ représente de l'hydrogène, et X³ représente de l'hydrogène, du t-butyle ou du méthyle.

31. Matière luminescente selon la revendication 3, dans laquelle B¹ représente de l'hydrogène, B² représente du 5'-phényl-m-terphényl-2-yle ou du 5'-phényl-m-terphényl-3-yle, chacun de X¹ et X⁵ représente de l'hydrogène ou du méthyle, chacun de X² et X⁴ représente de l'hydrogène, et X³ représente de l'hydrogène, du t-butyle ou du méthyle.

32. Matière luminescente selon la revendication 3, dans laquelle B¹ représente du m-quaterphényl-2-yle ou du m-quaterphényl-3-yle, B² représente de l'hydrogène, chacun de X¹ et X⁵ représente de l'hydrogène ou du méthyle, chacun de X² et X⁴ représente de l'hydrogène, et X³ représente de l'hydrogène, du t-butyle ou du méthyle.

33. Matière luminescente selon la revendication 3, dans laquelle B¹ représente de l'hydrogène, B² représente du m-quaterphényl-2-yle ou du m-quaterphényl-3-yle, chacun de X¹ et X⁵ représente de l'hydrogène ou du méthyle, chacun de X² et X⁴ représente de l'hydrogène, et X³ représente de l'hydrogène, du t-butyle ou du méthyle.

34. Matière luminescente selon la revendication 3, dans laquelle B¹ représente du 6-(m-terphényl-5'-yl)-2-naphthyle ou du 4-(m-terphényl-5'-yl)-1-naphthyle, B² représente de l'hydrogène, chacun de X¹ et X⁵ représente de l'hydrogène ou du méthyle, chacun de X² et X⁴ représente de l'hydrogène, et X³ représente de l'hydrogène, du t-butyle ou du méthyle.

35. Matière luminescente selon la revendication 3, dans laquelle B¹ représente de l'hydrogène, B² représente du 6-(m-terphényl-5'-yl)-2-naphthyle ou du 4-(m-terphényl-5'-yl)-1-naphthyle, chacun de X¹ et X⁵ représente de l'hydrogène ou du méthyle, chacun de X² et X⁴ représente de l'hydrogène, et X³ représente de l'hydrogène, du t-butyle ou du méthyle.

36. Dispositif électroluminescent organique, comprenant au moins une couche de transport de trous, une couche luminescente et une couche de transport d'électrons qui sont prises en sandwich entre une anode et une cathode sur un substrat, dans lequel la couche luminescente contient la matière luminescente selon la revendication 1.

37. Dispositif électroluminescent organique selon la revendication 36, dans lequel la couche luminescente contient au moins une impureté luminescente sélectionnée parmi des dérivés de pérylène, des dérivés de borane, des dérivés de styryle contenant de l'amine, des dérivés d'amine aromatique, des dérivés de coumarine, des dérivés de pyranne, des complexes d'iridium et des complexes de platine.

38. Dispositif électroluminescent organique selon la revendication 36, dans lequel la couche luminescente contient un dérivé de pérylène en tant qu'impureté luminescente.

39. Dispositif électroluminescent organique selon la revendication 36, dans lequel la couche luminescente contient un dérivé de borane en tant qu'impureté luminescente.

40. Dispositif électroluminescent organique selon la revendication 36, dans lequel la couche luminescente contient un dérivé de styryle contenant une amine en tant qu'impureté luminescente.

41. Dispositif électroluminescent organique selon la revendication 36, dans lequel la couche luminescente contient un dérivé d'amine aromatique en tant qu'impureté luminescente.

42. Dispositif électroluminescent organique selon la revendication 36, dans lequel la couche luminescente contient un dérivé de coumarine en tant qu'impureté luminescente.

43. Dispositif électroluminescent organique selon la revendication 36, dans lequel la couche luminescente contient un dérivé de pyranne en tant qu'impureté luminescente.

44. Dispositif électroluminescent organique selon la revendication 36, dans lequel la couche luminescente contient un complexe d'iridium en tant qu'impureté luminescente.

45. Dispositif électroluminescent organique selon la revendication 36, dans lequel la couche luminescente contient un complexe de platine en tant qu'impureté luminescente.

46. Dispositif électroluminescent organique selon la revendication 36, dans lequel la couche de transport d'électrons comprend un complexe métallique quinoléine.

47. Dispositif électroluminescent organique selon la revendication 36, dans lequel la couche de transport d'électrons comprend au moins l'un d'un dérivé de pyridine et d'un dérivé de phénanthroline.
